(19) Europäisches Patentamt / European Patent Office / Office européen des brevets

(11) **EP 3 906 835 B1**

(12) **EUROPÄISCHE PATENTSCHRIFT**

(45) Veröffentlichungstag und Bekanntmachung des Hinweises auf die Patenterteilung:
**29.11.2023  Patentblatt 2023/48**

(21) Anmeldenummer: **20172929.0**

(22) Anmeldetag: **05.05.2020**

(51) Internationale Patentklassifikation (IPC):
**A61B 1/00** (2006.01)　　　　**G02B 6/38** (2006.01)

(52) Gemeinsame Patentklassifikation (CPC):
**A61B 1/00096; A61B 1/00137; G02B 6/0008; G02B 23/2469**

(54) **LICHTAPPLIKATOR**

LIGHT APPLICATOR

APPLICATEUR DE LUMIÈRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**10.11.2021  Patentblatt 2021/45**

(73) Patentinhaber: **Richard Wolf GmbH**
**75438 Knittlingen (DE)**

(72) Erfinder:
• **Weber, Bernd-Claus**
**76228 Karlsruhe (DE)**
• **Sieber, Stephan**
**75438 Knittlingen-Freudenstein (DE)**

(74) Vertreter: **Patentanwälte Vollmann Hemmer Lindfeld**
**Partnerschaft mbB**
**Wallstraße 33a**
**23560 Lübeck (DE)**

(56) Entgegenhaltungen:
**EP-A1- 2 449 994**　　**CN-U- 202 590 069**
**US-A1- 2012 209 074**　　**US-B1- 9 488 347**

## Beschreibung

Die vorliegende Offenbarung betrifft einen Lichtapplikator zur Untersuchung und/oder Behandlung eines organischen Körpers, insbesondere zur photodynamischen Therapie (PDT) von pathologischem Gewebe.

Es ist bekannt, Endoskope dazu zu nutzen, um Videoaufnahmen vom Inneren eines menschlichen oder tierischen Körpers zu Zwecken der medizinischen Diagnose und/oder Therapie zu machen. Dabei ist es ein ständiges Bestreben, den Einführabschnitt von Endoskopen möglichst dünn auszugestalten, damit möglichst kleine Hohlräume eingesehen werden können und das Gewebe dabei möglichst wenig in Mitleidenschaft zu ziehen.

Endoskope werden allerdings nicht nur dazu genutzt, um Bild- oder Videoaufnahmen zu machen, sondern auch als Diagnose- oder Therapiemittel selbst eingesetzt. Beispielsweise für die Detektion und Lokalisierung von prä- und frühmalignem Gewebe kann eine Fluoreszenz-Endoskopie eingesetzt werden, bei der es nicht auf eine natürliche Echtfarb-Darstellung des Gewebes ankommt, sondern lediglich auf eine Fluoreszenzanregung, mit der sich pathologisches Gewebe von gesundem Gewebe unterscheiden lässt. Dabei kann das mittels Lichtstrahlung angeregte pathologische Gewebe selbst oder eine auf pathologisches Gewebe hinweisende Bakterienansammlung spezifisch fluoreszieren und so gegenüber dem umliegenden gesunden Gewebe erkennbar lokalisiert werden. Die Fluoreszenz-Endoskopie kann beispielsweise im Rahmen einer photodynamischen Diagnose (PDD) und/oder photodynamischen Therapie (PDT) mittels eines Photosensibilisators bzw. Markerstoffs (z.B. Chlorin e6) durchgeführt werden, der sich selektiv an pathologischem Gewebe anreichert.

Bei der photodynamischen Therapie (PDT) wird Licht mittels eines Lichtapplikators unmittelbar auf oder sogar in pathologisches Gewebe appliziert, um lichtinduziert die Bildung von Sauerstoffradikalen mittels des örtlich begrenzt angereicherten Photosensibilisators bzw. Markerstoffs zu fördern und dadurch das pathologische Gewebe, wie etwa einen Tumor, zu zerstören. Typischerweise wird dazu Laserlicht in einen Lichtleiter eingekoppelt und zum Gewebe geleitet. Wenn das pathologische Gewebe flächig auf einer äußeren Oberfläche, z. B. die Haut, oder einer inneren Oberfläche, z. B. Speiseröhreninnenfläche oder Darmwandung, angeordnet ist, dann kann das Therapielicht relativ einfach ausgekoppelt und auf die pathologische Gewebefläche gestrahlt werden. Erstreckt sich allerdings das pathologische Gewebe über ein Volumen, so kann wegen der begrenzten Eindringtiefe des Lichts in das Gewebe nicht immer ein Tumor von "außen" effektiv bestrahlt werden. In diesem Fall ist die PDT besonders effektiv, wenn das Licht vom Inneren des pathologischen Gewebevolumens aus möglichst isotrop abgestrahlt wird. Dazu muss der Lichtapplikator in das pathologische Gewebe eingestochen werden. Dies wird auch als interstitielle (durch innere Oberflächen) und/oder perkutane (durch die Haut) PDT bezeichnet.

In der EP 2 449 994 A1 ist beispielsweise beschrieben, wie ein Lichtapplikator in Form eines Laserlichtleiters in pathologisches Gewebe eingeschoben wird, nachdem der Weg für den Lichtleiter durch das Gewebe mit einer Nadel vorgestochen wurde.

Nachteilig an dieser bekannten Lösung ist einerseits, dass die Lichtauskopplung aus einem dünnen distalen Laserlichtleiterende nicht isotrop, sondern stark distalwärts gebündelt ist. Andererseits ist der dünne Laserlichtleiter selbst sehr biegsam, wodurch er mittels einer starren Kanalführung möglichst weit bis zum pathologischen Gewebe geführt sein muss und nicht weit aus der Kanalführung geschoben werden kann, ohne dass er sich verbiegt. Es müssen beim Einstechen je nach Eindringtiefe gegebenenfalls große Widerstände von zu durchdringendem gesundem und pathologischem Gewebe überwunden werden, sodass die mögliche Eindringtiefe bei der bekannten Lösung sehr begrenzt ist. Außerdem ist die bekannte Lösung relativ komplex und teuer, sodass sie nicht als Einweg-Artikel zum einmaligen Gebrauch realisierbar ist. Zudem besteht die Gefahr, dass die distale Spitze des Lichtapplikators abbricht und als schädlicher Fremdkörper operativ aus dem Körper des Patienten entfernt werden muss.

Daraus ergibt sich für die vorliegende Offenbarung die Aufgabe, einen Lichtapplikator bereitzustellen, der eine sichere interstitielle und/oder perkutane PDT oder PDD erlaubt.

Gemäß einem ersten Aspekt der vorliegenden Offenbarung wird zur Lösung dieses Problems ein Lichtapplikator zur Untersuchung und/oder Behandlung von einem organischen Körper bereitgestellt, wobei der Lichtapplikator einen minimalinvasiven Einführabschnitt aufweist, der sich entlang einer Längsrichtung erstreckt und an seinem distalen Ende eine LED aufweist, wobei am distalen Ende des Einführabschnitts eine sich zumindest teilweise distalwärts von der LED erstreckende und sich distalwärts verjüngende Nadelspitze mit einem lichttransparenten Streukörper zur Streuung des Lichts der LED angeordnet ist, wobei ein zumindest teilweise umfangseitig das distale Ende des Einführabschnitts und zumindest teilweise umfangseitig die Nadelspitze umgebendes Sicherheitselement mit dem Einführabschnitt und der Nadelspitze verbunden ist, wobei das Sicherheitselement zumindest abschnittsweise flexibel ist und im Falle eines zumindest teilweisen Abrisses oder Bruchs der Nadelspitze diese am distalen Ende des Einführabschnitts vor einem vollständigen Abreißen vom Lichtapplikator sichert.

Bei dem Sicherheitselement geht es also nicht um eine Erhöhung der Stabilität, sondern um eine Sicherheitsmaßnahme für den Fall, dass die auf die Nadelspitze einwirkenden Kräfte so groß sind, dass die Gefahr besteht, dass die Nadelspitze ggf. vollständig abreißt, auseinanderreißt oder abbricht und anschließend als schädlicher Fremdkörper operativ aus dem Körper des Patienten entfernt werden muss.

Optional kann das Sicherheitselement mittels einer ersten Verbindung mit dem distalen Ende des Einführabschnitts und mittels einer zweiten Verbindung mit der Nadelspitze jeweils stoff-, kraft-/reib- oder formschlüssig verbunden sein, wobei die zweite Verbindung einen axialen Abstand in Längsrichtung von der ersten Verbindung hat. Dadurch kann das Sicherheitselement zwischen den Verbindungen flexibel bleiben, um die Nadelspitze am distalen Ende des Einführabschnitts vor einem vollständigen Abreißen vom Lichtapplikator insgesamt und einem Zurückbleiben im Situs zu sichern. Die Nadelspitze kann dabei ggf. zwar vollständig vom distalen Ende des Einführabschnitts abreißen, bleibt aber aufgrund der zweiten Verbindung mit dem Sicherheitselement einerseits und aufgrund der ersten Verbindung des Sicherheitselements mit dem distalen Ende des Einführabschnitts andererseits mit dem Lichtapplikator insgesamt weiterhin verbunden. Die Flexibilität des Sicherheitselements und die daraus resultierende Nachgiebigkeit gegenüber äußeren Belastungen führt dazu, dass gemäß dem dritten Newtonschen Axiom auf die Nadelspitze nur noch eine reduzierte Kraft ausgeübt werden kann, wodurch ein Abreißen der Nadelspitze vom Applikator insgesamt verhindert wird. Optional kann dabei der Abstand mehr als 50% der axialen Länge des Sicherheitselements betragen. Vorzugsweise ist das Sicherheitselement also nur in axialen Endbereichen verklebt oder verlötet, und dazwischen nicht.

Optional kann das Sicherheitselement als geformter Metalldraht ausgeführt sein, der zumindest abschnittsweise formschlüssig mit der Nadelspitze verbunden ist. Beispielsweise kann der Metalldraht in die Nadelspitze eingegossen oder von dieser umgossen sein. Der Metalldraht verläuft dabei vorzugsweise gewinkelt durch die Nadelspitze, um somit einen Formschluss mit der Nadelspitze zu erzielen.

Optional kann das Sicherheitselement zumindest abschnittsweise axial verstreckt sein. Dies ist insbesondere dann von Vorteil, wenn abschnittsweise ein Formschluss zwischen Sicherheitselement und Nadelspitze bzw. Einführabschnitt hergestellt werden soll. Da eine axiale Verstreckung die Flexibilität beeinträchtigen kann, ist das Sicherheitselement vorzugsweise dort nicht axial verstreckt, wo es abschnittsweise flexibel bleiben soll, sondern nur dort, wo ein Formschluss mit Nadelspitze und/oder Einführabschnitt hergestellt werden soll. Zwischen der ersten Verbindung und der zweiten Verbindung kann das Sicherheitselement also unverstreckt und flexibel sein, wogegen es in den die erste und/oder zweite Verbindung umgebenden axialen Abschnitten axial verstreckt und dadurch weniger flexibel sein kann.

Optional kann das Sicherheitselement einen radial mittig angeordneten distalen Sicherheitselementabschnitt und einen radial außenseitig angeordneten proximalen Sicherheitselementabschnitt aufweisen, wobei der proximale Sicherheitselementabschnitt mit dem distalen Ende des Einführabschnitts verbunden ist und der distale Sicherheitselementabschnitt ein radial mittiges Verstärkungselement in Form einer Metallspitze der Nadelspitze bildet. Damit kann der distale Sicherheitselementabschnitt das distale Ende der Nadelspitze verstärken und/oder als distaler Nadelspitzenfortsatz fungieren.

Optional kann das Sicherheitselement in Form eines distal geschlossenen Schlauchs als auswechselbarer steriler Einwegartikel von distal über die gesamte Nadelspitze stülpbar sein. Der Schlauch kann dabei beispielsweise als Schrumpfschlauch thermisch aufschrumpfbar sein und/oder elastisch dehnbar über die gesamte Nadelspitze ziehbar sein.

Optional kann das Sicherheitselement in Form von mindestens einer schnur- oder fadenförmigen Komponente gebildet sein, die eine schlaufenartig geführte Überschusslänge aufweist. Vorzugsweise befindet sich die schlaufenartig geführte Überschusslänge in einem axial mittleren Abschnitt zwischen zwei verklebten axialen Endbereichen des Sicherheitselements. Die schlaufenartig geführte Überschusslänge ist vorzugsweise selbst nicht verklebt, sondern kann im Fall eines Bruchs oder Abrisses des Stabilisierungselements bei Krafteinwirkungen auf die Nadelspitze resilient nachgeben.

Zusätzlich kann optional ein umfangseitig das distale Ende des Einführabschnitts und die Nadelspitze zumindest teilweise umgebendes Stabilisierungselement mit dem Einführabschnitt und der Nadelspitze verbunden ist, wobei das Stabilisierungselement ein Material aufweist, das zumindest in Längsrichtung axial verstreckt ist. Das verstreckte Material kann dabei monoaxial, also vornehmlich in eine Richtung, oder bi-axial, d.h. planar in zwei Richtungen, verstreckt sein. Vorzugsweise handelt es sich bei dem verstreckten Material um einen Kunststoff, beispielsweise in Form einer Folie oder in der verstreckten Richtung ausgerichteter Fasern, wobei der Kunststoff eine gegenüber dem unverstreckten Kunststoff erhöhte Zugfestigkeit in der verstreckten Richtung aufweist. Eine Ausrichtung von Molekülketten in die verstreckte Richtung bewirkt dabei die Erhöhung der Zugfestigkeit im Kunststoff. Alternativ oder zusätzlich dazu kann Glas als verstrecktes Material dienen, das beispielsweise in Form von gezogenen Glasfasern in eine Richtung verstreckt ist. Dabei beruht die Erhöhung der Zugfestigkeit bei Glasfasern hauptsächlich darauf, dass die Größe von Fehlstellen, wie etwa Lufteinschlüsse, kleiner sind als der Faserdurchmesser, sodass die Wahrscheinlichkeit für Fehstellen mit dem Verstrecken sinkt. Im Falle von Metall als verstrecktem Material, das beispielsweise durch Walzen, Schmieden oder Kaltziehen verstreckt werden kann, beruht die erhöhte Zugfestigkeit auf einer Materialverdichtung und, insbesondere bei Metalldrähten, dem höheren Verhältnis von Oberfläche zum Volumen. In jedem Fall ist die Zugfestigkeit mindestens in die Längsrichtung gegenüber dem unverstreckten Material erhöht, vorzugsweise um ein Vielfaches. Das ist insbesondere wichtig, um den Lichtapplikator in seinen lateralen Ausmaßen einerseits so dünn wie möglich auszugestalten und anderseits stabil genug, um eine sichere interstitielle und/oder perkutane PDT oder PDD durchführen zu können. Der Durchmesser des Lichtapplikators kann hierbei 1 Millimeter oder weniger betragen, wobei das Stabilisierungselement eine Dicke von weniger als 200 Mikrometer haben kann. Durch das verstreckte Material kann das Stabilisierungselement besonders dünn und zugleich besonders zugfest in Längsrichtung ausgestaltet werden. Damit wird das Risiko verringert, dass zum einen die Nadelspitze in einem proximalen Nadelspitzenbereich abbricht und zum anderen die Nadelspitze als Ganzes vom distalen Ende des Einführabschnitts abreißt. Wirken Kräfte quer zur Längsrichtung auf die Nadelspitze ein, so kann das radial außen angeordnete und in axiale Richtung besonders zugfeste Stabilisierungselement diese Kräfte aufnehmen und einen Abbruch der Nadelspitze verhindern.

Optional kann das Stabilisierungselement zumindest teilweise das Sicherheitselement oder das Sicherheitselement zumindest teilweise das Stabilisierungselement umschließen. Vorzugsweise umschließt das Sicherheitselement das Stabilisierungselement, da das Stabilisierungselement dann möglichst großflächig mit seiner radialen Innenseite mit der Nadelspitze und dem distalen Ende des Einführabschnitts verklebt sein kann. Das Sicherheitselement kann dabei an seinen axialen Enden verklebt sein und einen axial zwischen den verklebten Enden angeordneten mittleren Abschnitt aufweisen, der nicht verklebt ist.

Optional können das Stabilisierungselement und das Sicherheitselement abwechselnd in einer zur Längsrichtung orthogonalen Umfangsrichtung das distale Ende des Einführabschnitts und die Nadelspitze zumindest teilweise umgreifen. Dadurch können sich in Umfangsrichtung das Stabilisierungselement und das Sicherheitselement ergänzen, ohne sich radial überlappen zu müssen, sodass damit die laterale Ausdehnung reduziert werden kann.

Optional kann das Stabilisierungselement und/oder das Sicherheitselement das distale Ende des Einführabschnitts und die Nadelspitze zumindest teilweise schlauchförmig umgreifen. Das hat den Vorteil, dass bei Abbruch und/oder Abriss der Nadelspitze etwaige Bruchstücke oder Splitter innerhalb des Stabilisierungselements und/oder des Sicherheitselements verbleiben und nicht in den Körper des Patienten eindringen. Beispielsweise kann das Stabilisierungselement und/oder das Sicherheitselement ein Schrumpfschlauch sein, der zumindest teilweise außenseitig auf das distale Ende des Einführabschnitts und die Nadelspitze thermisch aufgeschrumpft ist.

Optional kann das Stabilisierungselement und/oder das Sicherheitselement zumindest teilweise aus einer lichttransparenten Kunststofffolie bestehen. Dies ist vorteilhaft, da die Nadelspitze vorzugsweise nahezu vollständig aus einem Streukörper besteht, der mit möglichst großer Effizienz Licht der LED abstrahlt. Würde die Nadelspitze teilweise von einem nicht transparenten Stabilisierungselement und/oder Sicherheitselement umgeben, so beeinträchtigte dies signifikant die Effizienz der Lichtabstrahlung.

Optional kann das Stabilisierungselement und/oder das Sicherheitselement eine axiale Verbindungsstelle zwischen dem distalen Ende des Einführabschnitts und der Nadelspitze vollständig umlaufend umschließen. Besonders im Bereich der Verbindungsstelle mit einer stoffschlüssigen Klebeverbindung zwischen Nadelspitze und dem distalen Ende des Einführabschnitts kann das Risiko für einen Bruch oder Abriss erhöht sein, sodass dort abbrechende Bruchstücke oder Splitter vom Stabilisierungselement und/oder Sicherheitselement umschlossen bleiben und nicht in den Körper des Patienten eindringen.

Optional kann das Stabilisierungselement und/oder das Sicherheitselement eine radiale Dicke aufweisen, die weniger als 20% des Durchmessers des Einführabschnitts beträgt. Die radiale Dicke kann also jeweils sehr dünn sein und beispielsweise unter 300 Mikrometer, vorzugsweise unter 50 Mikrometer oder sogar unter 10 Mikrometer liegen.

Optional kann das Stabilisierungselement einen distalen Stabilisierungselementabschnitt und/oder das Sicherheitselement einen distalen Sicherheitselementabschnitt aufweisen, wobei der distale Stabilisierungselementabschnitt und/oder der distale Sicherheitselementabschnitt an der Nadelspitze zumindest teilweise radial zuläuft. Dadurch kann ein Formschluss erzielt werden, der das Risiko eines Abrisses verringert. Dabei kann beispielsweise der distale Stabilisierungselementabschnitt und/oder der distale Sicherheitselementabschnitt zumindest teilweise in die Nadelspitze eingossen bzw. von dieser umspritzt sein. Alternativ oder zusätzlich dazu kann sich der distale Stabilisierungselementabschnitt und/oder der distale Sicherheitselementabschnitt distalwärts mit der Nadelspitze korrespondierend verjüngen. Es ist dabei vorteilhaft, wenn der distale Stabilisierungselementabschnitt und/oder der distale Sicherheitselementabschnitt Material aufweist, das in Umlaufrichtung verstreckt ist. Dadurch dehnt er sich weniger leicht radial auf und bildet einen entsprechend stärkeren Formschluss mit der Nadelspitze.

Optional kann das Stabilisierungselement und/oder das Sicherheitselement zumindest teilweise das distale Ende des Einführabschnitts und zumindest einen proximalen Nadelspitzenabschnitt strumpfförmig umgreifen, wobei das Stabilisierungselement und/oder das Sicherheitselement axiale Durchbrechungen aufweist, durch welche ein distaler Nadelspitzenabschnitt mit dem proximalen Nadelspitzenabschnitt stoffschlüssig verbunden ist. Dies kann einen besonders starken Formschluss zwischen dem Stabilisierungselement bzw. Sicherheitselement und der Nadelspitze schaffen. Es kann dann ggf. auf eine distale Verklebung des Stabilisierungselements und/oder des Sicherheitselements verzichtet werden.

Optional kann das Stabilisierungselement axial in Längsrichtung eine höhere Zugfestigkeit aufweisen als in eine zur Längsrichtung orthogonale Umfangsrichtung. Beispielsweise kann das verstreckte Material dabei monoaxial verstreckt sein.

Optional kann das Stabilisierungselement zumindest teilweise aus einem kristallisierten oder teilkristallisierten Polymer bestehen, dessen Molekülketten eine Vorzugsausrichtung in Längsrichtung aufweisen und in einer Ebene quer zur Vorzugsausrichtung enger bzw. dichter nebeneinander liegen. Die Herstellung eines solchen Polymers ist relativ einfach und günstig, wobei das Verstrecken in Längsrichtung eine besonders hohe Zugfestigkeit in Längsrichtung bewirkt.

Optional kann die Zugfestigkeit des Stabilisierungselements in Längsrichtung mindestens doppelt so hoch sein wie die Zugfestigkeit des Stabilisierungselements in einer zur Längsrichtung orthogonalen Umfangsrichtung. Vorzugsweise ist die Zugfestigkeit in Längsrichtung um ein Vielfaches höher als in einer zur Längsrichtung orthogonalen Umfangsrichtung. In der Umfangsrichtung kann das Stabilisierungselement zwar zusätzlich verstreckt sein, muss es aber nicht.

Optional kann das Stabilisierungselement als ein proximalseitiger Nadelspitzenhülsenfortsatz ausgebildet sein, wobei der Nadelspitzenhülsenfortsatz einen Faser-Kunststoff-Verbund mit axial verstreckten Fasern aufweist, wobei die Fasern eine Vorzugsausrichtung in Längsrichtung aufweisen. Der Volumenanteil an verstreckten Fasern kann hierbei 50% oder mehr betragen. Die radiale Dicke des Nadelspitzenhülsenfortsatzes kann beispielsweise im Bereich von 50 Mikrometer bis 300 Mikrometer liegen.

Optional kann das Stabilisierungselement über mehr als 50% seiner axialen Länge radial innenseitig verklebt sein. Vorzugsweise kann das Stabilisierungselement mit größtmöglicher radialer Innenfläche mit der Nadelspitze und dem distalen Ende des Einführabschnitts verklebt sein.

Gemäß einem zweiten Aspekt der vorliegenden Offenbarung wird ein Verfahren zum Herstellen eines Lichtapplikators bereitgestellt mit den Schritten:

- Bereitstellen eines Lichtapplikators mit einem minimalinvasiven Einführabschnitt, der sich entlang einer axialen Längsrichtung erstreckt und an seinem distalen Ende eine LED aufweist, wobei am distalen Ende des Einführabschnitts eine sich zumindest teilweise distalwärts von der LED erstreckende und sich distalwärts verjüngende Nadelspitze mit einem lichttransparenten Streukörper zur Streuung des Lichts der LED angeordnet ist,
- Aufziehen eines flexiblen Sicherheitselements in Form eines Schrumpfschlauchs über die Nadelspitze und zumindest teilweise über das distale Ende des Einführabschnitts,
- Aufschrumpfen des Sicherheitselements auf das distale Ende des Einführabschnitts, und
- Axiales Verstrecken eines die sich verjüngende Nadelspitze umgebenden axialen Abschnitts des Sicherheitselements.

Optional kann während des axialen Verstreckens das Sicherheitselement zumindest abschnittsweise am distalen Ende des Einführabschnitts festgehalten werden und somit dort unverstreckt und flexibel bleiben.

Optional kann während des axialen Verstreckens oder in einem separaten Schritt das Sicherheitselement zumindest abschnittsweise erwärmt werden, wodurch der die verjüngende Nadelspitze umgebende axiale Abschnitt des Sicherheitselements an die sich verjüngende Nadelspitze thermisch fixiert wird.

Die Begriffe "distalwärtig" bzw. "proximalwärtig" sollen hierin eine relative Position bedeuten, die sich distal bzw. proximal von einem Anwender des Systems als Bezugsposition befindet. Die Begriffe "distalseitig" bzw. "proximalseitig" sollen hierin entsprechend eine Position an einer distalen bzw. proximalen Seite eines Gegenstands bedeuten. Die Begriffe "distalwärts" bzw. "proximalwärts" soll hierin entsprechend eine Richtung bedeuten, die sich nach distal bzw. proximal erstreckt.

Die Offenbarung ist nachfolgend anhand von in den Zeichnungen dargestellten Ausführungsbeispielen näher erläutert. Es zeigen:

Fig. 1 eine schematische Darstellung einer beispielhaften Ausführungsform eines hierin offenbarten Lichtapplikatorsystems;

Fig. 2 eine schematische Darstellung einer anderen beispielhaften Ausführungsform eines hierin offenbarten Lichtapplikatorsystems;

Fig. 3 eine schematische Darstellung eines Beispiels eines Lichtapplikators einer beispielhaften Ausführungsform eines hierin offenbarten Lichtapplikatorsystems;

Fig. 4 eine schematische Darstellung eines anderen Beispiels eines Lichtapplikators einer beispielhaften Ausführungsform eines hierin offenbarten Lichtapplikatorsystems;

Fig. 5 eine schematische Darstellung einer weiteren beispielhaften Ausführungsform eines hierin offenbarten Lichtapplikatorsystems;

Fig. 6 eine schematische Darstellung einer weiteren beispielhaften Ausführungsform eines hierin offenbarten Lichtapplikators;

Fig. 7 eine schematische Darstellung einer weiteren beispielhaften Ausführungsform eines hierin offenbarten Lichtapplikators;

Fig. 8    eine schematische Darstellung einer weiteren beispielhaften Ausführungsform eines hierin offenbarten Lichtapplikators;

Fig. 9a,b    einen schematischen Längsschnitt und eine schematische Draufsicht auf einen ersten Leiter mit distalseitiger LED eines möglichen Lichtapplikators einer beispielhaften Ausführungsform des hierin offenbarten Lichtapplikatorsystems;

Fig. 10a,b    einen schematischen Längsschnitt und eine schematische Draufsicht auf einen ersten Leiter mit distalseitiger LED eines möglichen Lichtapplikators einer beispielhaften anderen Ausführungsform des hierin offenbarten Lichtapplikatorsystems;

Fig. 11a-d    schematische Darstellungen eines Einführabschnitts vier weiterer beispielhafter Ausführungsformen eines hierin offenbarten Lichtapplikators;

Fig. 12a-c    schematische Darstellungen der distalen Spitze eines möglichen Lichtapplikators einer beispielhaften Ausführungsform des hierin offenbarten Lichtapplikatorsystems;

Fig. 13    eine schematische Darstellung der distalen Spitze eines möglichen Lichtapplikators einer beispielhaften Ausführungsform des hierin offenbarten Lichtapplikatorsystems während des Betriebs;

Fig. 14    eine schematische Darstellung der distalen Spitze eines möglichen Lichtapplikators einer beispielhaften Ausführungsform des hierin offenbarten Lichtapplikatorsystems mit zwei verschiedenen Detailvarianten;

Fig. 15    eine schematische Darstellung der distalen Spitze eines möglichen Lichtapplikators einer anderen beispielhaften Ausführungsform des hierin offenbarten Lichtapplikatorsystems mit vier verschiedenen Detailvarianten;

Fig. 16    eine schematische Darstellung der distalen Spitze eines möglichen Lichtapplikators einer beispielhaften Ausführungsform des hierin offenbarten Lichtapplikatorsystems beim Einstich in die Haut eines Patienten;

Fig. 17    eine schematische Darstellung der distalen Spitze eines möglichen Lichtapplikators einer anderen beispielhaften Ausführungsform des hierin offenbarten Lichtapplikatorsystems beim Einstich in die Haut eines Patienten;

Fig. 18    eine schematische Darstellung der distalen Spitze eines möglichen Lichtapplikators einer anderen beispielhaften Ausführungsform des hierin offenbarten Lichtapplikatorsystems beim Einstich in die Haut eines Patienten;

Fig. 19    einen schematischen Querschnitt der distalen Spitze eines möglichen Lichtapplikators einer beispielhaften Ausführungsform des hierin offenbarten Lichtapplikatorsystems mit zwei verschiedenen Detailvarianten;

Fig. 20    eine schematische Darstellung eines Verstärkungselements einer distalen Spitze eines möglichen Lichtapplikators einer beispielhaften Ausführungsform des hierin offenbarten Lichtapplikatorsystems mit drei verschiedenen Detailvarianten;

Fig. 21    eine schematische Darstellung der distalen Spitze eines möglichen Lichtapplikators einer beispielhaften Ausführungsform des hierin offenbarten Lichtapplikatorsystems beim Einstich in die Haut eines Patienten;

Fig. 22    einen schematischen Querschnitt der distalen Spitze eines möglichen Lichtapplikators einer beispielhaften Ausführungsform des hierin offenbarten Lichtapplikatorsystems mit drei verschiedenen Detailvarianten;

Fig. 23    eine schematische Darstellung eines Verstärkungselements einer distalen Spitze eines möglichen Lichtapplikators einer beispielhaften Ausführungsform des hierin offenbarten Lichtapplikatorsystems;

Fig. 24    eine schematische Darstellung der distalen Spitze eines möglichen Lichtapplikators einer anderen beispielhaften Ausführungsform des hierin offenbarten Lichtapplikatorsystems mit Hautöffnung beim Einstich in fünf verschiedenen Phasen;

Fig. 25    eine schematische Darstellung der distalen Spitze eines möglichen Lichtapplikators einer anderen beispielhaften Ausführungsform des hierin offenbarten Lichtapplikatorsystems mit zwei verschiedenen Detailvarianten;

Fig. 26 eine schematische Darstellung der distalen Spitze eines möglichen Lichtapplikators einer anderen beispielhaften Ausführungsform des hierin offenbarten Lichtapplikatorsystems mit zwei verschiedenen Detailvarianten;

Fig. 27 eine schematische Darstellung der distalen Spitze eines möglichen Lichtapplikators einer anderen beispielhaften Ausführungsform des hierin offenbarten Lichtapplikatorsystems;

Fig. 28a,b schematische Längsschnitte der distalen Spitze eines Ausführungsbeispiels eines hierin offenbarten Lichtapplikators;

Fig. 29 einen schematischen Längsschnitt der distalen Spitze eines Ausführungsbeispiels eines hierin offenbarten Lichtapplikators;

Fig. 30a-c einen schematischen Längsschnitt und zweite alternative Draufsichten (ohne Nadelspitze) der distalen Spitze eines weiteren Ausführungsbeispiels eines hierin offenbarten Lichtapplikators;

Fig. 31 einen schematischen Längsschnitt der distalen Spitze eines weiteren Ausführungsbeispiels eines hierin offenbarten Lichtapplikators;

Fig. 32a,b schematische Längsschnitte der distalen Spitze eines weiteren Ausführungsbeispiels eines hierin offenbarten Lichtapplikators;

Fig. 33a,b schematische Längsschnitte der distalen Spitze eines weiteren Ausführungsbeispiels eines hierin offenbarten Lichtapplikators;

Fig. 34a,b schematische Längsschnitte der distalen Spitze eines weiteren Ausführungsbeispiels eines hierin offenbarten Lichtapplikators;

Fig. 35a,b schematische Längsschnitte der distalen Spitze eines weiteren Ausführungsbeispiels eines hierin offenbarten Lichtapplikators;

Fig. 36a-c schematische Längsschnitte der distalen Spitze eines weiteren Ausführungsbeispiels eines hierin offenbarten Lichtapplikators;

Fig. 37a-d schematisch Verfahrensschritte zum beispielhaften Anbringen eines Sicherheitselements an einem hierin offenbarten Lichtapplikator; und

Fig. 38a-c schematische Längsschnitte der distalen Spitze eines weiteren Ausführungsbeispiels eines hierin offenbarten Lichtapplikators.

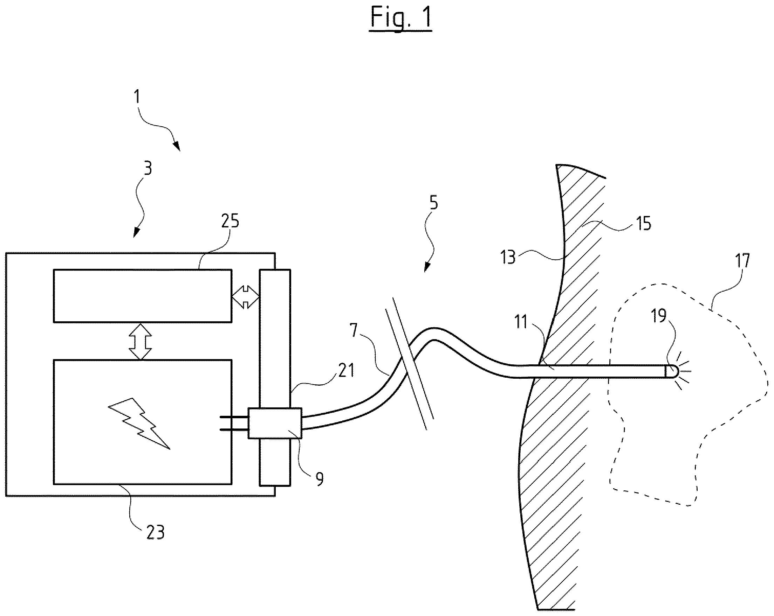

Fig. 1 zeigt ein Lichtapplikatorsystem 1 mit einer Lichtapplikatorbetriebseinheit 3 und einem auswechselbaren Lichtapplikator 5. Der Lichtapplikator 5 besteht im Wesentlichen aus einem flexiblen Kabelabschnitt 7, der sich von einem proximalseitigen Stecker 9 zu einem distalseitigen Einführabschnitt 11 erstreckt. Der Einführabschnitt 11 ist im Gegensatz zum Kabelabschnitt 7 der Abschnitt des Lichtapplikators 5, der dazu vorgesehen ist, in organisches Gewebe eines Körpers 13 eingestochen zu werden. Der Lichtapplikator 5 ist hier für die perkutane PDT geeignet und im PDT-Betrieb gezeigt. Der Einführabschnitt 11 ist hier als starrer Nadelabschnitt ausgestaltet, der beispielsweise CT- oder ultraschallunterstützt von außen durch die Haut 13 eines Patienten und darunterliegendes gesundes Gewebe 15 mit der distalen Spitze in pathologisches Körpergewebe 17, z. B. einen malignen Tumor, gestochen ist.

An der distalen Spitze weist der Einführ- bzw. Nadelabschnitt 11 eine distalseitige LED 19 zur In-Situ-Erzeugung von Anregungslicht für die PDT auf. Wird beispielsweise Chlorin e6 als Sensibilisator für die PDT eingesetzt, kann die dazu speziell ausgelegte LED 19 im Wellenlängenbereich von 660-670 nm Anregungslischt aussenden. Das Chlorin e6, das sich als Sensibilisator zuvor im pathologischen Körpergewebe 17 selektiv angereichert hat, produziert durch die Lichteinwirkung Sauerstoffradikale, die das pathologische Körpergewebe 17 zerstören. Da sich das Chlorin e6 nicht im gesunden Gewebe 15 anreichert und die applizierten Lichtmengen so gering sind, dass sie keine thermisch bedingten Schäden am Gewebe herbeiführen, bleibt das gesunde Gewebe 15 vom Licht im Wesentlichen unbeeinflusst.

Die LED 19 wird über einen elektrischen Leiter im Kabelabschnitt 7 und Einführabschnitt 11 von der Lichtapplikatorbetriebseinheit 3 mit Strom versorgt. Der proximalseitige Stecker 9 des Lichtapplikators 5 ist dazu in einen Anschluss 21 an der Lichtapplikatorbetriebseinheit 3 gesteckt. Zur Stromversorgung des Lichtapplikators 5 weist die Lichtapplikatorbetriebseinheit 3 ein elektrisches Versorgungsmodul 23 auf, das dazu eingerichtet ist, am Anschluss 21 einen bestimmten Betriebsstrom für den anschließbaren Lichtapplikator 5 bereitzustellen. Außerdem weist die Lichtapplikatorbetriebseinheit 3 ein Steuerungsmodul 25 auf, das dazu eingerichtet ist, den Stecker 9 zu identifizieren und das Versorgungsmodul 23 anzuweisen einen entsprechenden Betriebsstrom bereitzustellen.

Der Anschluss 21 kann beispielsweise eine Steckerleiste mit einer Mehrzahl von Steckplätzen sein, wobei als Identifikationsmittel in Form einer mechanischen Kennung nur bestimmte Steckerformen in bestimmte Steckplätze passen, sodass bei eingestecktem Stecker 9 eindeutig bestimmt ist, welcher Typ von Lichtapplikator 5 sich an einem Steckplatz befindet. Das Steuerungsmodul 25 kann dann festgelegte Betriebsparameter (Strom, Spannung, Betriebsdauer, usw.) pro Steckplatz vorsehen. Alternativ oder zusätzlich kann der Applikatortyp als Identifikationsmittel in Form einer signalbasierten Kennung vom Steuerungsmodul 25 auslesbar im Stecker 9 gespeichert sein und das Versorgungsmodul 23 entsprechend angesteuert werden. Bei einer signalbasierten Kennung kann sich der Lichtapplikator 5 selbst aktiv beim Steuerungsmodul 25 "anmelden" und identifizieren und/oder passiv vom Steuerungsmodul 25 abgefragt werden. Es kann auch eine vorhergehende Benutzung des Lichtapplikators 5 im Steuerungsmodul 25 und/oder im Stecker 9 abgespeichert sein, sodass nur eine einmalige Verwendung des Lichtapplikators 5 erlaubt werden kann.

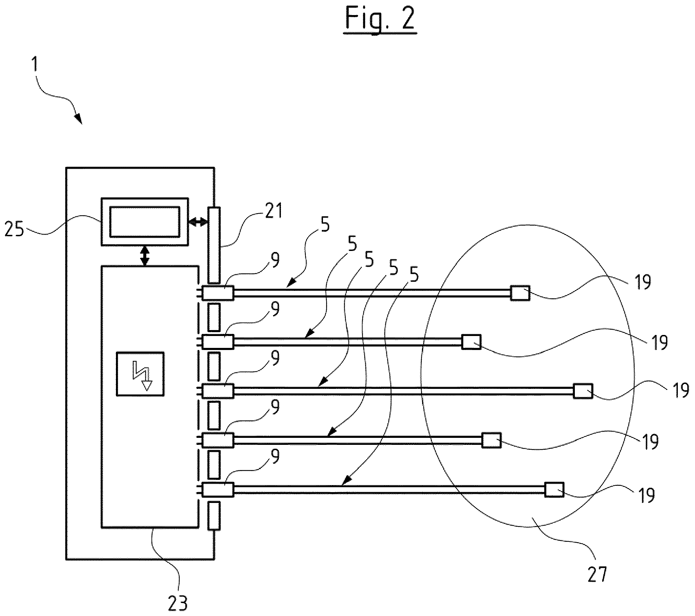

In Fig. 2 weist das Lichtapplikatorsystem 1 eine Mehrzahl von Lichtapplikatoren 5 auf, die zeitgleich an einer Mehrzahl an Anschlüssen 21 angeschlossen sind, um gemeinsam für eine PDT eines ganzen Organs 27 verwendet zu werden. Die Lichtapplikatoren 5 können alle gleichen Typs oder zumindest teilweise unterschiedlichen Typs sein. Die Lichtapplikatoren 5 sind an unterschiedlichen Stellen durch die Haut und mit unterschiedlichen Einstichtiefen verteilt in das Organ 27 eingestochen, sodass die LEDs 19 das Organ möglichst homogen mit Anregungslicht ausleuchten. Dies ist vor allem bei großen und/oder nicht genau lokalisierbaren Tumoren sinnvoll. Da gesundes Gewebe keinen Schaden durch die PDT erleidet, ist es sinnvoll, großzügig ein Volumen oder ein ganzes Organ um den Tumor auszuleuchten, um vor allen Dingen das Risiko zu verringern, dass okkultes pathologisches Gewebe untherapiert bleibt. Die einzelnen Lichtapplikatoren 5 können mit bestimmtem Typ, an bestimmter Position, mit bestimmtem Einstichwinkel und -tiefe beispielsweise mittels einer auf die Haut geklebten oder anderweitig fixierten Hilfsschablone bzw. Template festgelegt sein. Die entsprechende Zuordnung der Lichtapplikatoren 5 zu den Anschlüssen 21 kann über die Identifikationsmittel festgelegt sein, sodass für eine festgelegte Art der PDT an einem bestimmten Organ 27 automatisch an jedem Anschluss 21 von dem Steuerungsmodul 25 die richtigen Betriebsparameter bereitgestellt werden. Das Fehlerrisiko bei einer komplexen Einstellung einer Mehrzahl an Lichtapplikatoren 5 kann somit verringert werden.

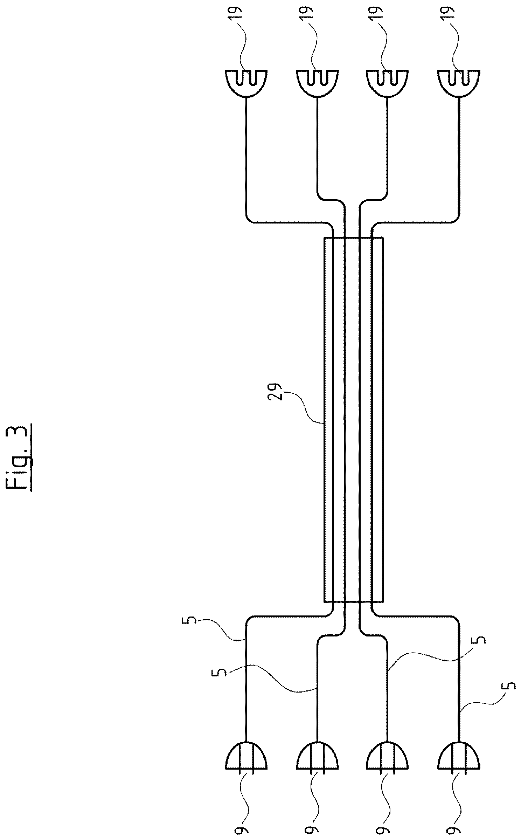

In Fig. 3 ist schematisch gezeigt, dass eine Mehrzahl von Lichtapplikatoren 5 für die interstitielle oder perkutane PDT im flexiblen Kabelabschnitt 7 zumindest abschnittsweise gebündelt sein kann, beispielsweise in Form eines mehradrigen Flachbandkabels 29. Die Lichtapplikatoren 5 können allerdings auch gebündelt oder einzeln durch einen oder mehrere Arbeitskanäle eines Endoskops oder Trokars geführt sein.

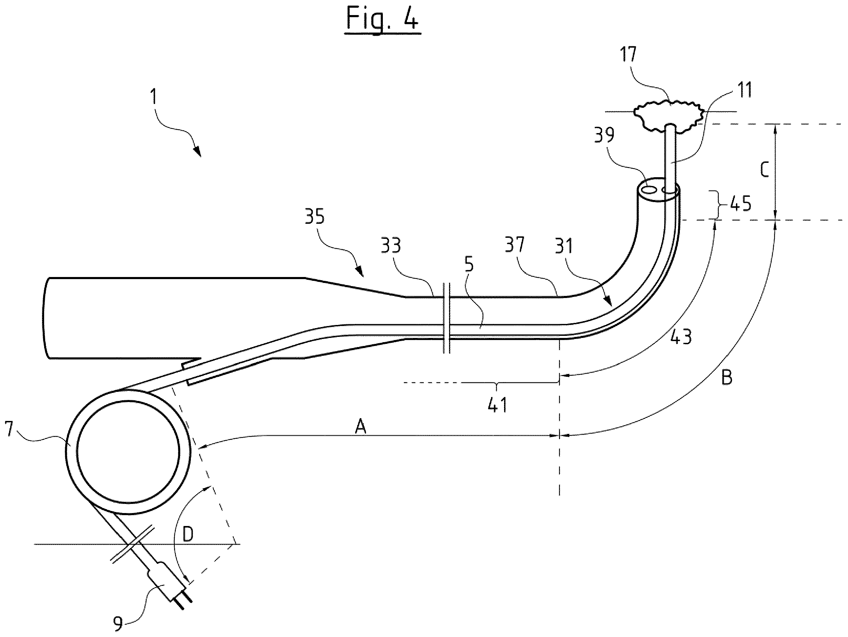

In Fig. 4 ist eine Ausführungsform eines Lichtapplikatorsystems 1 gezeigt, bei dem ein Lichtapplikator 5 für die interstitielle PDT durch einen Arbeitskanal 31 im Schaft 33 eines Schaftinstruments 35 in Form eines Endoskops geführt ist. Der Schaft 33 weist einen Einführabschnitt 37 auf, der dazu bestimmt ist, in den Körper eines Patienten eingeführt zu werden. Optional kann am distalen Ende des Einführabschnitt 37 ein Bildsensor 39 angeordnet sein, um ein Bild vom Inneren des Körpers des Patienten aufnehmen zu können. Der Einführabschnitt 37 weist einen ersten Schaftabschnitt 41, einen zweiten Schaftabschnitt 43 und einen dritten Schaftabschnitt 45 auf, wobei der erste Schaftabschnitt 41 proximal vom zweiten Schaftabschnitt 43 und der dritte Schaftabschnitt 45 distal vom zweiten Schaftabschnitt 43 verläuft. Der Schaft 33 ist im zweiten Schaftabschnitt 43 abwinkelbar und/oder weniger biegesteif als der erste und/oder dritte Schaftabschnitt 41, 45. Damit kann das distale Ende des Einführabschnitts 37 an verwinkelte Bereiche des Köperinneren, wie etwa Nierenkelche oder stark verzweigte Atemwege, minimalinvasiv platziert werden.

Entsprechend der Schaftabschnitte 41, 43, 45 weist auch der Lichtapplikator 5 verschiedene Lichtapplikatorabschnitte auf, nämlich einen ersten Lichtapplikatorabschnitt A, einen zweiten Lichtapplikatorabschnitt B und einem dritten Lichtapplikatorabschnitt C, wobei der erste Lichtapplikatorabschnitt A proximal vom zweiten Lichtapplikatorabschnitt B und der dritte Lichtapplikatorabschnitt C distal vom zweiten Lichtapplikatorabschnitt B verläuft. Der zweite Lichtapplikatorabschnitt B ist weniger biegesteif als der erste A und/oder dritte Lichtapplikatorabschnitt C. Der zweite Lichtapplikatorabschnitt B ist zumindest teilweise im zweiten Schaftabschnitt 43 angeordnet, wenn die distalseitige LED 19 des Lichtapplikators 5 am distalseitigen Ende des Einführabschnitts positioniert ist. Die Lichtapplikatorabschnitte A, B, C sind also in der Länge abgestimmt auf den Schaft 33 des Endoskops 35, damit einerseits der Lichtapplikator 5 dort biege- und torsionssteif genug ausgestaltet ist, um das distale Ende des Lichtapplikators 5 steuern zu können und andererseits dort flexibel genug ausgestaltet ist, um die Abwinkelbarkeit des Schafts im zweiten Schaftabschnitt 43 möglichst wenig zu beeinträchtigen. Der Lichtapplikator 5 hat in diesem Ausführungsbeispiel noch einen optionalen vierten Lichtapplikatorabschnitt D im proximalen flexiblen Kabelabschnitt 7, der proximalseitig vom ersten Lichtapplikatorabschnitt A und in gewissem Radius aufwickelbar außerhalb vom Endoskop 35 verläuft. Der distale Einführabschnitt 11 des Lichtapplikators 5 kann am distalen Ende des Arbeitskanals 31 herausragen und für die interstitielle PDT in pathologisches Gewebe 17 eingestochen werden. Der distale Einführabschnitt 11 des Lichtapplikators 5 kann dazu flexibel oder zusammen mit einer distalen Nadelspitze als Nadelabschnitt relativ biegesteif sein. Ein biegesteifer Einführ- bzw. Nadelabschnitt 11 kann die Eindringtiefe in das Gewebe erhöhen, wogegen ein flexibler Einführabschnitt 11 besser durch einen abgewinkelten Arbeitskanal 31 schieb- oder ziehbar ist.

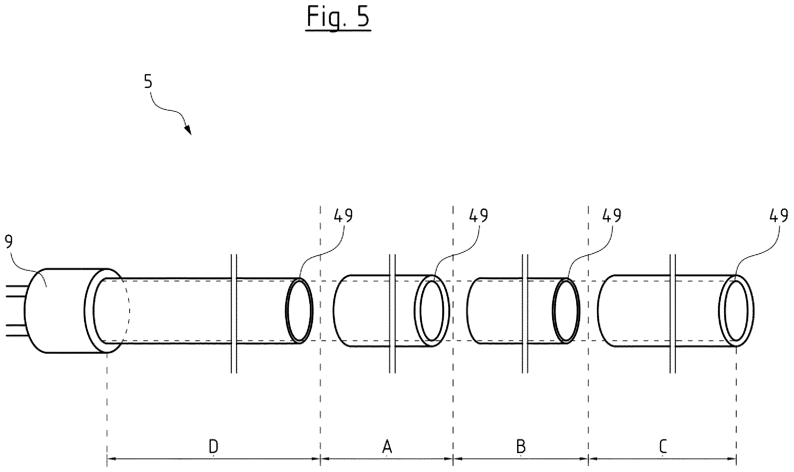

Wie in Fig. 5 gezeigt, ist der Lichtapplikator 5 im zweiten Lichtapplikatorabschnitt B dünner als in den Lichtapplikatorabschnitten A und C ausgestaltet, da eine geringere Biege- und Torsionssteifigkeit für kleine Abwinkelradien gebraucht wird. In den Lichtapplikatorabschnitten A und C ist er dicker, wo eine höhere Biege- und Torsionssteifigkeit vorteilhaft für die Handhabung ist. Es kann dabei die radiale Ausdehnung des elektrischen Leiters selbst und/oder seine isolierende Ummantelung den Erfordernissen für die lokale Biege- und Torsionssteifigkeit angepasst sein. Der Lichtapplikator 5 muss dabei nicht als Ganzes unterschiedlich dick in den verschiedenen Lichtapplikatorabschnitten A-D sein, sondern es kann beispielsweise nur ein versteifender Mantel unterschiedlich dick in den verschiedenen Lichtapplikatorabschnitten A-D sein. Eine sehr einfache Lösung ist es, eine radiale Ummantelung 49 als elektrischer Isolator und/oder Wärmedämmung abschnittsweise als Schläuche mit unterschiedlicher Wandstärke und/oder in unterschiedlicher Anzahl als zumindest teilweise miteinander überlappende Schläuche auszubilden und somit unterschiedliche Biege- und Torsionssteifigkeiten in den Lichtapplikatorabschnitten A-D zu erzielen. Die Schläuche können dabei als Schrumpfschläuche ausgeführt sein. Die Wandstärke der Ummantelung kann vergleichsweise dünn ausgestaltet werden, da die durch die Ummantelung bewirkte Erhöhung der Biegesteifigkeit mit dem Flächenträgheitsmoment $I_r$ korreliert. Beispielsweise bei einem Kreisring als Querschnittsform der Ummantelung gilt $I_r = \frac{\pi}{4}(R^4 - r^4)$ , sodass die Biegesteifigkeit mit der vierten Potenz des Radius skaliert, wobei R der Außenradius der Ummantelung und r der Innenradius der Ummantelung ist. Für einen Ummantelungsquerschnitt mit quadratischem Kastenprofil mit Außenbreite A und Innenbreite a gilt entsprechend $I_r = \frac{1}{12}(A^4 - a^4)$ . Selbst geringste Unterschiede der Dicke der Ummantelung zwischen den Lichtapplikatorabschnitten A bis D im Bereich von $10\,\mu m$ können daher einen signifikanten Einfluss auf die Biege- und Torsionssteifigkeit bewirken.

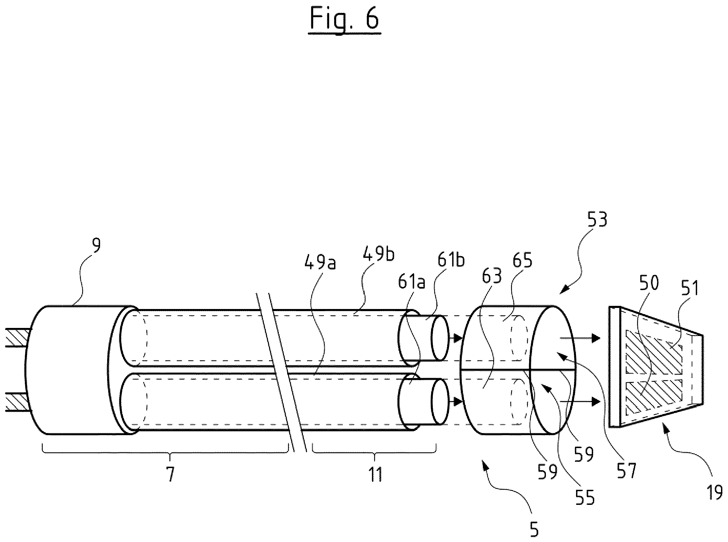

Fig. 6 zeigt eine Ausführungsform des Lichtapplikators 5, bei dem ein sogenannter Flip-Chip als LED 19 zum Einsatz kommt, bei dem sowohl der erste elektrische Kontakt 50 (hier Anodenkontakt) als auch der zweite elektrische Kontakt 51 (hier Kathodenkontakt) proximalseitig angeordnet sind. Der LED-Chip 19 ist hier beispielsweise mit einer dünnen Leitkleberschicht (nicht abgebildet) auf einem Adapterstück 53 aufgeklebt, das hinsichtlich seiner lateralen Abmessungen so ausgeführt ist, dass eine großflächige Anlage des LED-Chips 19 ermöglicht wird und damit ein guter proximalwärtiger Wärmefluss von der LED 19 zum Adapterstück 53 erzielt wird.

Das Adapterstück 53 kann dabei wie in Fig. 6 aus zwei Metallhälften 55, 57 bestehen, die jeweils mit den Kontakten 50, 51 der LED in elektrisch leitendem Kontakt stehen und gegeneinander mittels einer dünnen Isolator-Schicht 59 elektrisch isoliert sind. Alternativ kann das Adapterstück 53 beispielsweise als einstückiges Keramikstück mit metallischen Durchführungen ausgeführt sein. Die LED wird über die Länge des Lichtapplikators 5 mit einem elektrischen Leiter 61 mit Strom versorgt, wobei hier der elektrische Leiter 61 als Zwillingslitze 61a,b ausgeführt ist, wobei jeweils eine der Zwillingslitzen 61a,b der Stromzuführung und die anderer der Zwillingslitzen 61b,a der Stromabführung dient. Das Adapterstück 53 weist proximalseitig Kontaktaufnahmen 63, 65 auf, mit welchen das distale Ende des elektrischen Leiters 61 elektrisch leitend verbindbar ist. Der elektrische Leiter 61 kann zumindest abschnittsweise flexibel oder starr ausgestaltet sein. Ob flexibel oder starr, es ist für den proximalwärtigen Wärmeabfluss in jedem Fall von Vorteil, wenn der elektrische Leiter 61 einen möglichst großen Querschnittsanteil, z.B. mindestens 70%, am Lichtapplikator 5 hat, da der elektrische Leiter 61 ein besserer Wärmeleiter ist als eine den elektrischen Leiter 61 umgebende isolierende Ummantelung 49a,b.

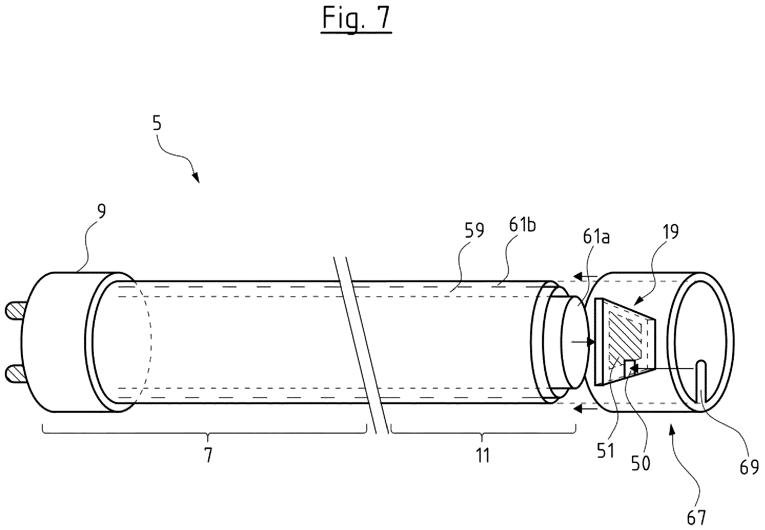

Im Gegensatz zum Ausführungsbeispiel gemäß Fig. 6 ist in Fig. 7 ein Lichtapplikator 5 gezeigt mit einem LED-Chip 19, der einen proximalseitig großflächigen zweiten elektrischen Kontakt 51 und einen distalseitigen relativ kleinflächigen ersten elektrischen Kontakt 50 aufweist. Der elektrische Leiter 61 ist hier als Koaxialkabel ausgeführt, wobei dessen Seele 61a distalseitig mittels Leitkleber oder Lötzinn mit dem proximalseitigen zweiten elektrischen Kontakt 51 der LED 19 leitend verbunden ist. Für einen möglichst guten proximalwärtigen Wärmeabfluss von der LED 19 über die Seele 61a hat diese einen relativ großen Querschnittsflächenanteil am elektrische Leiter 61 bzw. am gesamten Lichtapplikator 5, z.B. mindestens 70%. Ein Außenleiter 61b und eine zwischen Außenleiter 61b und Seele 61a angeordnete Isolator-Schicht 59 sind entsprechend jeweils möglichst dünn ausgeführt. Nach außen hin ist der Außenleiter 61b von ebenfalls mindestens einer möglichst dünnen isolierenden Ummantelung (nicht in Fig. 7 gezeigt) umgeben. Wie in Fig. 5 kann über die Dicke und/oder Schichtenzahl der Ummantelung abschnittsweise die Biegesteifigkeit des Lichtapplikators 5 definiert sein. Die Seele 61a kann als relativ starrer massiver Draht oder als flexibles Litzenbündel ausgestaltet sein.

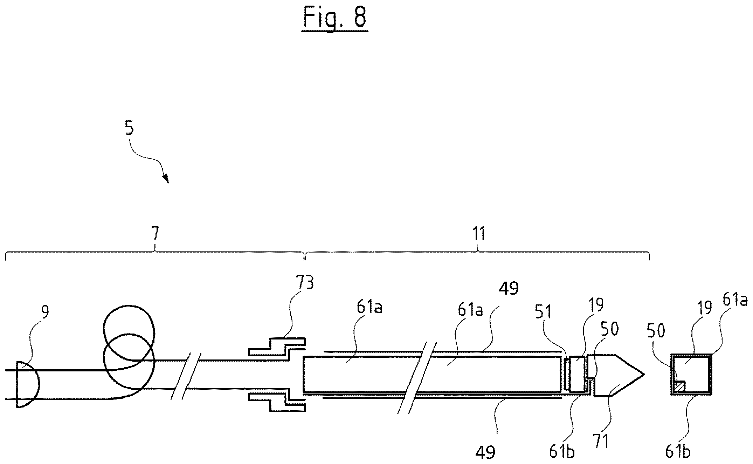

Der Außenleiter 61b in Fig. 7 dient als Rückleiter, der über eine distalseitige metallische Hülse 67, die über die LED 19 gestülpt ist, mit dem ersten elektrischen Kontakt 50 der LED 19 elektrisch leitend verbunden ist. Für den elektrischen Kontakt zwischen dem ersten elektrischen Kontakt 50 und der Hülse 67 ragt ein Kontaktbügel 69 der Hülse 67 radial nach innen. Wenngleich nicht in allen Figuren gezeigt, ist bei allen Ausführungsformen vorteilhafterweise distal von der LED eine lichtstrahlformende Komponente angeordnet, um eine gewünschte Abstrahlcharakteristik zu erzielen. Beispielsweise kann für die PDT eine isotrope Abstrahlung gewünscht sein, sodass ein Streukörper 71 (siehe Figuren 8-10 und 13-27) die lichtstrahlformende Komponente bilden kann. Falls eine Fokussierung des Lichts gewünscht ist, kann eine Linse als lichtstrahlformende Komponente distalseitig vom LED-Chip 19 angeordnet sein.

In Fig. 8 ist eine Ausführungsform des Lichtapplikators 5 gezeigt, die besonders vorteilhaft für die perkutane PDT ist. In Fig. 8 ist rechts separat eine Draufsicht auf die LED 19 gezeigt. Der proximale Kabelabschnitt 7 des Lichtapplikators 5 mit proximalseitigem Stecker 9 zum Anschluss an eine Lichtapplikatorbetriebseinheit 3 ist flexibel und weist distalseitig einen lösbaren Verbindungsanschluss 73 für einen relativ starren distalen Nadelabschnitt 11 auf, wobei der Nadelabschnitt 11 in diesem Ausführungsbeispiel den Einführabschnitt 11 samt Streukörper 71 als Nadelspitze beschreibt. Der Nadelabschnitt 11 dient also hier ganz oder teilweise als Einführabschnitt zum Einstechen in Gewebe eines Körpers. Der Nadelabschnitt 11 erhält seine Biegesteifigkeit hauptsächlich durch einen zentralen, massiven, starren, stabförmigen ersten elektrischen Leiter 61a. Analog zu Fig. 7 ist distalseitig am ersten elektrischen Leiter 61a eine LED 19 verbunden. Aus der Draufsicht auf die LED 19 rechts in Fig. 8 wird deutlich, dass der elektrische Leiter 61a wie die LED 19 einen im Wesentlichen quadratischen Querschnitt hat, der nur wenig größer ist als die LED 19. Der Querschnitt der momentan am Markt erhältlichen oder produzierbaren kleinstmöglichen LED 19 setzt hier eine untere Grenze für die Miniaturisierung des Lichtapplikators 5, der möglichst minimalinvasiv, also dünn, ausgestaltet ist und somit möglichst wenig über die lateralen Abmessungen der LED 19 hinausragen sollte. So können momentan Lichtapplikatorquerschnitte von deutlich weniger als 1 mm² erzielt werden, beispielsweise 0,25 mm² und weniger.

Ein zweiter elektrischer Leiter 61b kann als Rückleiter in Form einer gegen den ersten elektrischen Leiter 61a isolierten sehr dünnen Flexplatine oder eines Lackdrahts an einer Seite des ersten elektrische Leiters 61a entlanggeführt und an seinem distalen Ende L-förmig gefaltet oder gebogen sein, um den distalseitigen ersten elektrischen Kontakt 50 der LED 19 zu kontaktieren. Die metallische Hülse 67 aus Fig. 7 kann dann in beiden Fällen entfallen. Die Leiter 61a, 61b sind hier mit einer dünnen Ummantelung 49 ummantelt, die vorzugsweise aus Kunststoff als Schrumpfschlauch biokompatibel ausgebildet ist und das Gleitverhalten des Nadelabschnitts 11 durch Köpergewebe verbessert. Außerdem dient die Ummantelung 49 als elektrische Isolierung und Wärmedämmung nach außen.

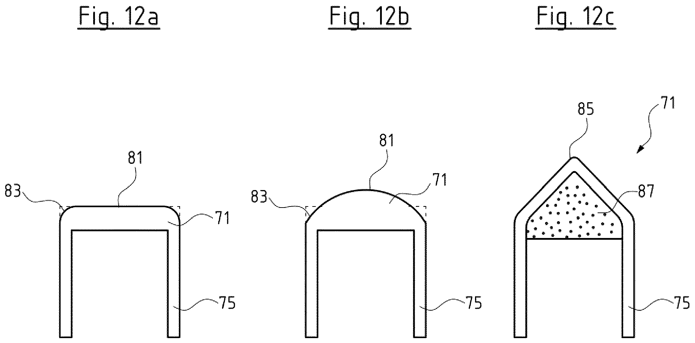

In Fig. 8 ist eine distal von der LED 19 angeordnete lichtstrahlformende Komponente in Form eines lichttransparenten Streukörpers 71 gezeigt, der als eine sich distalwärts verjüngende Nadelspitze des Nadelabschnitts 11 fungiert. In Figuren 12 bis 37 sind vorteilhafte Ausgestaltungen der Nadelspitze näher gezeigt.

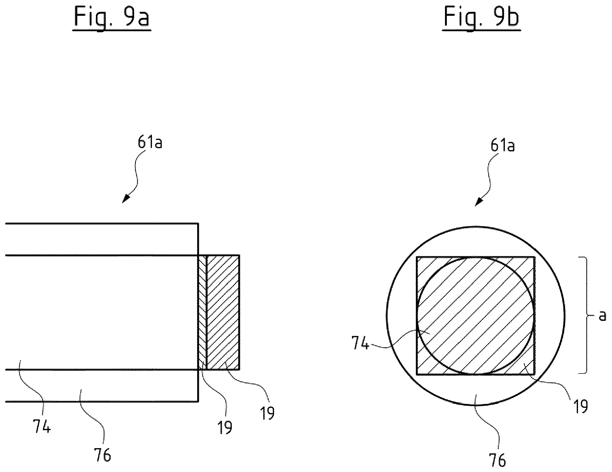

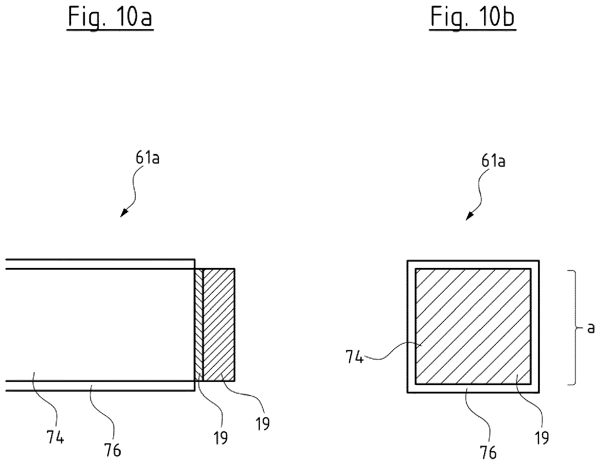

Um bei Querschnitten von unter 1 mm² eine hohe Biegesteifigkeit des ersten elektrischen Leiters 61a zu erzielen, ist es vorteilhaft, ein Material mit möglichst hohem Elastizitätsmodul als erster elektrischer Leiter zu verwenden. Beispielsweise Stahl hat mit über 200 MPa einen hohen Elastizitätsmodul. Allerdings hat Stahl den Nachteil, dass seine Wärmeleitfähigkeit deutlich unter 100 W/(Km) bei 20°C liegt, üblicherweise im Bereich von 50 W/(Km) bei 20°C. Da der erste elektrische Leiter 61a nicht nur biegesteif, sondern auch möglichst gut die Abwärme der LED proximalwärts ableiten soll, wäre zur Wärmeleitung ein erster elektrischer Leiter 61a aus Kupfer oder Silber mit einer Wärmeleitfähigkeit von mehr als 400 W/(Km) bei 20°C sinnvoll, oder zumindest aus Aluminium mit einer Wärmeleitfähigkeit von mehr als 200 W/(Km) bei 20°C. Allerdings haben Kupfer, Aluminium und Silber mit unter 100 MPa einen wesentlich geringeren Elastizitätsmodul als Stahl. Zur Erzielung einer hohen Biegesteifigkeit bei gleichzeitig hoher Wärmeleitfähigkeit ist der elektrische Leiter 61a, wie in Figuren 9a,b und Fig. 10a,b gezeigt, mit einem Kupferkern 74 und einem Stahlmantel 76 ausgeführt. Der Stahlmantel 76 kann relativ dünnwandig im Vergleich zum Radius des Kupferkerns 74 sein, da der Versteifungseffekt wie oben beschrieben in vierter Potenz mit dem Radius skaliert. Gleichzeitig ist die proximalwärtige Wärmeleitung besser je größer der Querschnitt des Kupferkerns 74 ist. Für eine möglichst gute Wärmeableitung ist der Durchmesser des Kupferkerns 74 an die lateralen Abmessungen der LED 19 angepasst. In Figuren 9a,b ist der Querschnitt des Leiters 61a rund und die LED 19 in etwa quadratisch mit einer Seitenlänge a, sodass der Durchmesser des Kupferkerns 74 in etwa a beträgt. Der Stahlmantel 76 ist hier mindestens ( $\sqrt{2} - 1$ ) a dick, damit die Ecken der LED 19 nicht lateral über den Leiter 61a hinausragen. In Figuren 10a,b ist der Querschnitt des Leiters 61a wie die LED 19 in etwa quadratisch, wobei der Querschnitt des Kupferkerns 74 in etwa dem Querschnitt der LED 19 entspricht. Der Stahlmantel 76 ist hier wesentlich dünner als in Figuren 9a,b, z.B. weniger als 0,15 · a dick.

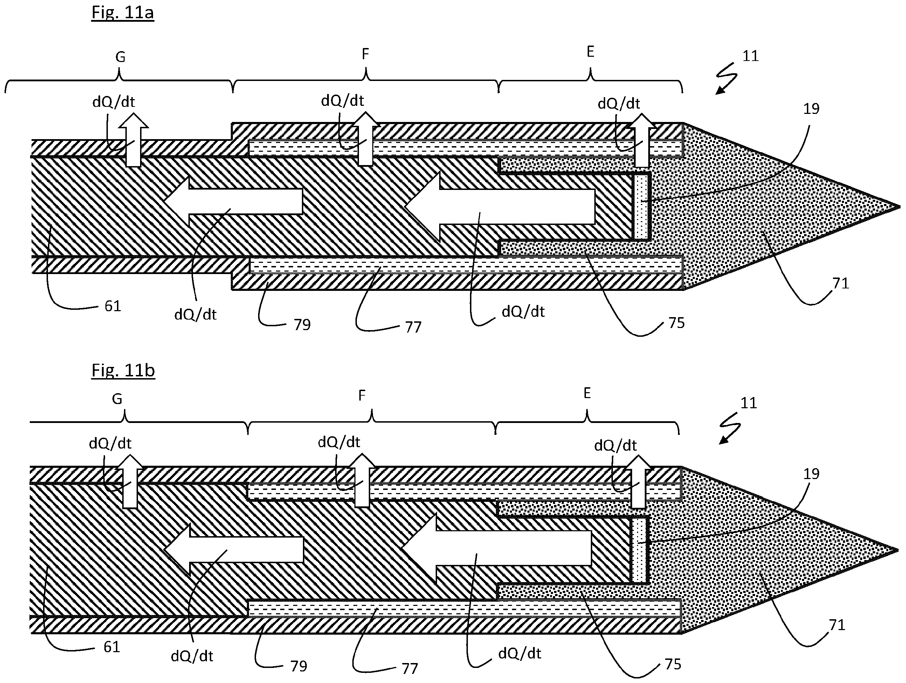

In Figuren 11a und 11b wird das Wärmemanagement im Einführabschnitt 11 des Lichtapplikators 5 deutlich. Der Einführabschnitt 11 kann hier starr oder flexibel sein, also der elektrische Leiter 61 als massiver Stab oder als flexibles Litzenbündel ausgebildet sein. Es besteht eine technische Herausforderung darin, bei kleinen Querschnitten, insbesondere Querschnitten von unter 1 mm², die Abwärme der LED 19 einerseits so gut abzuleiten, dass die LED 19 keinen Schaden nimmt und auch mit voranschreitender Einschaltzeit der LED die Höhe der abgegebenen optischen Leistung aufrecht erhalten werden kann, da mit ansteigender LED-Temperatur die abgegebene optische Leistung absinkt. Anderseits darf das umgebende Körpergewebe nicht so stark erhitzt werden, dass es thermisch bedingten Schaden nimmt. Eine zu starke Wärmedämmung entlang der Achse des Einführabschnitts 11 schadet der LED 19 und eine zu schwache Wärmedämmung schadet aufgrund eines extrem inhomogenen radialen Wärmeflusses und eines vor allen Dingen im Bereich der LED 19 sehr stark überhöhten radialen Wärmeflusses vom Applikator an das umgebende Gewebe dort dem Gewebe. Zur Lösung dieses Problems nimmt die radiale Wärmedämmung proximalwärts von der LED 19 ab. In den gezeigten Ausführungsbeispielen geschieht dies stufenweise zwischen mehreren Wärmedämmungsabschnitten E, F, G. In einem ersten distalen Wärmedämmungsabschnitt E, der die LED 19 radial umgibt, ist die radiale Wärmedämmung am stärksten, sodass in diesem Bereich des Einführabschnitts 11, also im Bereich der LED 19 als Wärmequelle, wo der Wärmefluss dQ/dt im Lichtapplikator 5 insgesamt sehr hoch ist, weil dort fast noch keine Wärme an die äußere Umgebung abgegeben werden konnte, durch eine gute radiale Wärmedämmung der radiale Wärmefluss dQ/dt vom Lichtapplikator 5 zum Gewebe auf ein moderates Maß gedämpft wird, um das umliegende Gewebe thermisch nicht zu schädigen. Dazu weist die Wärmedämmung im distalen Wärmedämmungsabschnitt E drei Schichten auf.

Radial ganz innen wirkt als erste Wärmedämmschicht 75 im ersten distalen Wärmedämmungsabschnitt E ein sich hülsenförmig um die LED 19 erstreckender proximaler Abschnitt 75 der Nadelspitze 71, die distal von der LED 19 einen lichttransparenten Streukörper bildet. Der Streukörper 71 bewirkt eine möglichst isotrope Abstrahlung des Lichts in einen möglichst großen Raumwinkel. Die erste Wärmedämmschicht 75 weist wie der Streukörper 71 selbst einen Kunststoff, beispielsweise Epoxidharz, als Hauptbestandteil auf. Kunststoffe sind vorteilhafterweise durch eine vergleichsweise geringe Wärmeleitfähigkeit gekennzeichnet. Als zweite Wärmedämmschicht umgibt im ersten distalen Wärmedämmungsabschnitt E ein erster Schrumpfschlauch 77 aus Kunststoff, beispielsweise Polyethylenterephthalat (PET), die erste Wärmedämmschicht 75. Als dritte Wärmedämmschicht umgibt im ersten distalen Wärmedämmungsabschnitt E ein zweiter Schrumpfschlauch 79 aus Kunststoff, beispielsweise Polyethylenterephthalat (PET), die zweite Wärmedämmschicht 77.

Vom ersten distalen Wärmedämmungsabschnitt E erstreckt sich proximalwärts ein zweiter Wärmedämmungsabschnitt F, der aus der zweiten Wärmedämmschicht 77 und der dritten Wärmedämmschicht 79 besteht. Die erste Wärmedämmschicht 75 aus dem proximalen Hülsenabschnitt der Nadelspitze 71 erstreckt sich nicht bis in den zweite Wärmedämmungsabschnitt F. Dadurch kann der Leiter 61 die Wärme besser radial abgeben als im ersten Wärmedämmungsabschnitt E, sodass ein Wärmestau vermieden wird und ein proximalwärtiger Wärmeabfluss von der LED 19 durch den Leiter 61 gewährleistet bleibt. Vom zweiten distalen Wärmedämmungsabschnitt F erstreckt sich proximalwärts ein dritter Wärmedämmungsabschnitt G, der nur aus der dritten Wärmedämmschicht 79 besteht. Die zweite Wärmedämmschicht 77 erstreckt sich nicht bis in den dritten Wärmedämmungsabschnitt G. Dadurch kann der Leiter 61 die Wärme noch besser radial abgeben als im zweiten Wärmedämmungsabschnitt F, sodass auch hier ein Wärmestau vermieden wird und ein proximalwärtiger Wärmeabfluss von der LED 19 durch den Leiter 61 gewährleistet bleibt. Im Ergebnis bewirkt die proximalwärts von der LED 19 abnehmende radiale Wärmedämmung eine Verteilung des radialen Wärmeflusses über die Länge des Einführabschnitt 11, sodass der longitudinale Temperaturgradient $\Delta T/\Delta L$ am Außenradius der dritten Wärmedämmschicht 79 über die Wärmedämmungsabschnitte E, F, G möglichst gering ist und somit gewebeschädigende Temperaturspitzen vermieden werden. Wie durch die Größe der weißen Blockpfeile in Fig. 11a,b angedeutet, wird der Unterschied im radialen Temperaturgradient $\Delta T/\Delta r$ zwischen den Wärmedämmungsabschnitten E, F, G durch die unterschiedliche Dicke der radialen Wärmedämmung und die daraus resultierenden unterschiedlichen radialen thermischen Widerstände der Wärmedämmungsabschnitte E, F, G möglichst ausgeglichen, sodass die jeweils durch die Wärmedämmungsabschnitte E, F, G radial nach außen übertragene Wärmeleistung dQ/dt möglichst gleich ist.

In den Figuren 11a und 11b wird die Stärke der Wärmedämmung hauptsächlich über die Gesamtdicke der Wärmedämmung bestimmt. Wie in Fig. 11a gezeigt, kann der Lichtapplikator 5 daher proximalwärts dünner ausgestaltet werden. Dies ist allerdings kein großer Vorteil, da der maximale Querschnitt des Lichtapplikators 5 durch die erste Wärmedämmschicht E mit drei Wärmedämmschichten bestimmt ist. In Fig. 11b ist gezeigt, dass sich entsprechend der proximalwärts abnehmenden Dicke der Wärmedämmung der Querschnitt des Leiters 61 vergrößern kann, sodass der Querschnitt des Lichtapplikator 5 über die Wärmedämmungsabschnitte E, F, G hinweg im Wesentlichen konstant ist. Damit wird die proximalwärtige Wärmeleitung dQ/dt durch den Leiter 61 entsprechend verbessert.

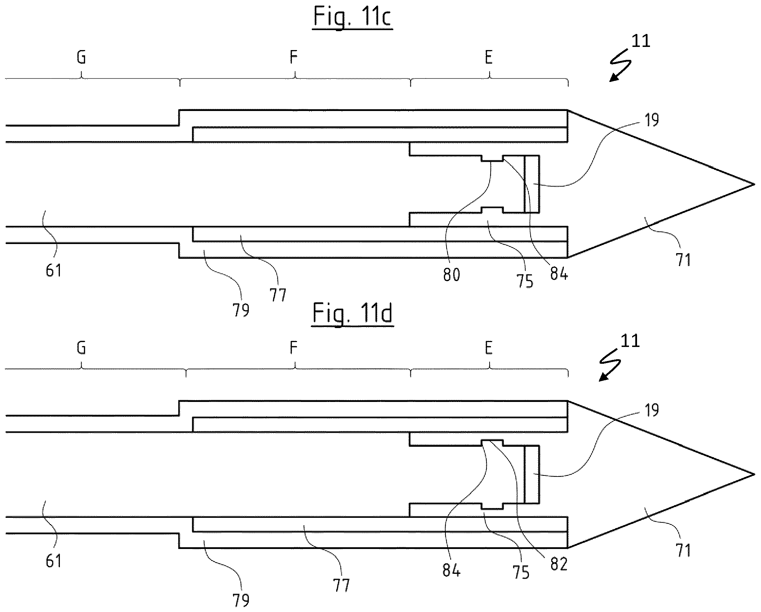

In den Figuren 11c und 11d ist gezeigt, wie eine zusätzliche Verbesserung der Befestigungssicherheit für die Nadelspitze 71 erreicht werden kann. Der Leiter 61 weist dazu in einem distalen Endabschnitt außenseitig mindestens eine Vertiefung 80 (siehe Fig. 11c) und/oder Aufweitung 82 (siehe Fig. 11d) auf, beispielweise als lokale Querschnittsverjüngung und/oder -vergrößerung. Dadurch wird erreicht, dass der hülsenförmige proximale Abschnitt 75 der Nadelspitze 71 mit dem ersten Leiter 61 einen Formschluss bildet. Die Nadelspitze 71 kann als Kunststoffgussteil oder als Kunststoffspritzgussteil ausgebildet sein, das beispielsweise durch Umspritzen oder Umgießen mit der außenseitigen Vertiefung 80 oder Aufweitung 82 des Leiters 61 einen Formschluss mittels eines Hinterschnitts 84 bilden kann.

In den Figuren 12 bis 27 sind verschiedene Ausgestaltungen der Nadelspitze bzw. des Streukörpers 71 gezeigt. Figuren 12a-c zeigen jeweils einen prinzipiellen hülsenförmigen Aufbau einer strahlformenden Komponente distalseits der LED (nicht gezeigt). In Fig. 12a ist die Nadelspitze im Wesentlichen topfförmig mit ebener Stirnfläche 81 und leicht abgerundeten Kanten 83. Die Nadelspitze in Fig. 12a ist daher relativ stumpf. Dies kann vorteilhaft sein, wenn kein Gewebe geschnitten, sondern nur weggedrückt werden soll. Für die perkutane PDT ist solch eine Nadelspitze allerdings wegen des hohen Widerstands beim Einstechen weniger geeignet. In Fig. 12b ist die Stirnfläche 81 komplett abgerundet, was einerseits den Widerstand beim Einstechen gegenüber der ebenen Stirnfläche 81 aus Fig. 12a verringert und andererseits einen Linseneffekt erzielt. In Fig. 12c verjüngt sich die Nadelspitze distalwärts kegelförmig zu einer relativ stumpfen Spitze 85. Das Kegelvolumen ist hier zum Großteil mit einem lichtstreuenden Material 87 ausgefüllt.

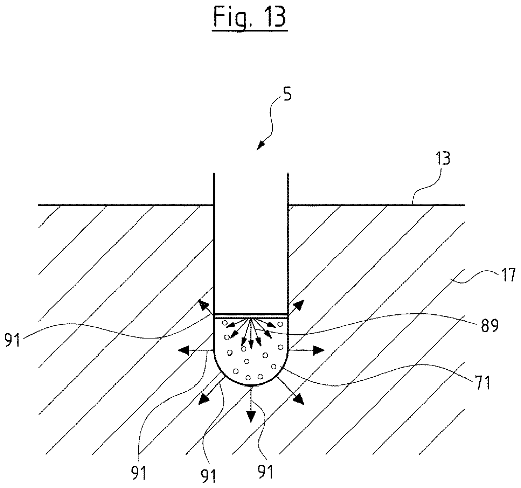

Fig. 13 zeigt einen perkutan (durch die Haut 13) in pathologisches Gewebe 17 eingestochenen Lichtapplikator 5 mit distalseitiger Nadelspitze, die als kuppelförmiger Streukörper 71 ausgebildet ist. Der Streukörper 71 streut das von der LED 19 gemäß der Abstrahlcharakteristik eines Lambert'schen Strahlers abgestrahlte Licht 89 derart, dass das den Streukörper 71 verlassende Licht 91 möglichst isotrop in einen möglichst gro-βen Raumwinkel abgestrahlt wird. Das vom Streukörper 71 abgestrahlte Licht hat dadurch teilweise auch proximalwärts gerichtete Richtungskomponenten.

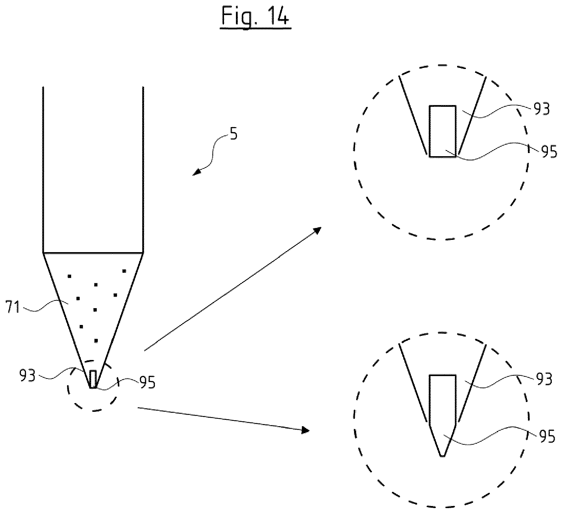

Bei der interstitiellen oder perkutanen PDT soll die mechanische Einwirkung auf Haut 13 und Gewebe 15, 17, insbesondere auf gesundes Gewebe 15, das ggf. auf dem Weg zum pathologischen Gewebe 17 passiert werden muss, möglichst minimalinvasiv sein. Es ist das vorrangige Ziel, dass der Einstich keine dauerhaften Schädigungen der Haut 13 und des gesunden Gewebes 15 herbeiführt. Außerdem soll der Einstich möglichst schnell verheilen und keine oder kleinstmögliche Narben hinterlassen. Zu diesem Zweck kann es vorteilhaft sein, die Spitze des Lichtapplikators 5 besonders spitz und/oder scharf auszugestalten. Dabei kann es allerdings technisch schwierig sein, die distale Spitze und/oder die Kante scharf genug aus dem lichttransparenten Material des Streukörpers 71 herstellen zu können. Beispielsweise ist Epoxidharz wie die meisten anderen Kunststoffe relativ weich und kann sich beim Einstich an der Spitze und/oder Kante verbiegen, wenn diese entsprechend spitz bzw. scharf ausgeführt ist. Härteres lichttransparentes Material wie etwa Quarzglas ist zwar biegesteifer, jedoch ist die Bearbeitung deutlich aufwändiger und dadurch mit wesentlich höheren Kosten verbunden. Außerdem sind härtere transparente Werkstoffe wie beispielsweise Quarzglas spröde, sodass das Risiko von Absplitterungen sehr hoch wäre. Zur Lösung dieses Problems weist, wie in Fig. 14 gezeigt, ein Nadelspitzenendbereich 93 des beispielweise kegel- oder pyramidenförmigen Streukörpers 71 ein Verstärkungselement 95 auf. Das Verstärkungselement 95 ist aus hartem Metall, wie etwa Stahl, und zumindest teilweise in ein relativ weiches Streukörpermaterial, wie etwa Epoxidharz eingebettet. Im Ausführungsbeispiel von Fig. 14 ist das Verstärkungselement 95 als relativ kurzer Dorn ausgeführt, der zur Verstärkung des Nadelspitzenendbereichs 93 zur Erhöhung sowohl der Biegesteifigkeit als auch der Biegefestigkeit ganz eingebettet sein kann (siehe Detailansicht rechts oben in Fig. 14) oder zur Schärfung der Nadelspitze nun, im Vergleich mit der Detailansicht rechts oben, spitz zulaufend distal aus dem Streukörper 71 herausragt (siehe Detailansicht rechts unten in Fig. 14). Die Querschnittsfläche des Verstärkungselements 95 ist relativ klein, damit es distalwärtig möglichst wenig Schatten wirft. Dazu ist es auch von Vorteil, das Verstärkungselement 95 möglichst kurz auszugestalten, wohingegen der Versteifungseffekt des Nadelspitzenendbereich 93 größer ist bei längerer Ausführung des Dorns 95.

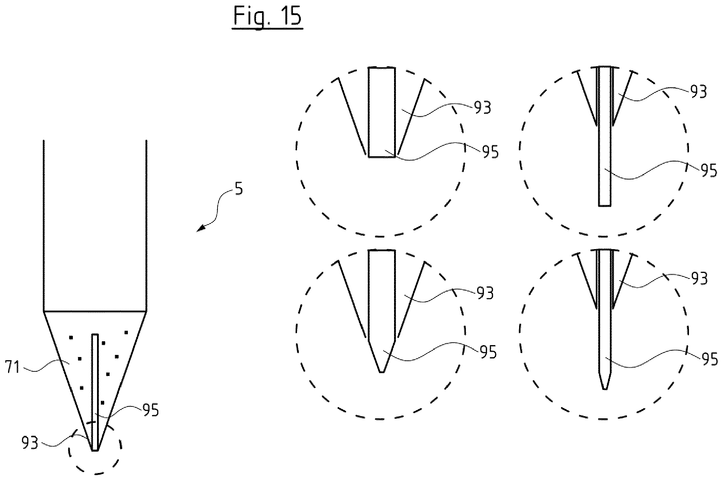

In Fig. 15 ist solch eine längere Ausführung des Dorns 95 als Verstärkungselement gezeigt. Bei dünner Ausführung ist der etwas größere Schattenwurf im Vergleich zum kurzen Dorn 95 aus Fig. 14 vernachlässigbar. Außerdem wird das Ausmaß des Schattenwurfs durch die lichtstreuende Wirkung des Streukörpers 71 eingeschränkt. Durch die tiefere Einbettung des Dorn 95 in den Streukörper 71 ist der Dorn 95 besser stabilisiert und kann ggf. weiter distalwärts aus dem Streukörper 71 herausragen (siehe Detailansichten rechts in Fig. 15).

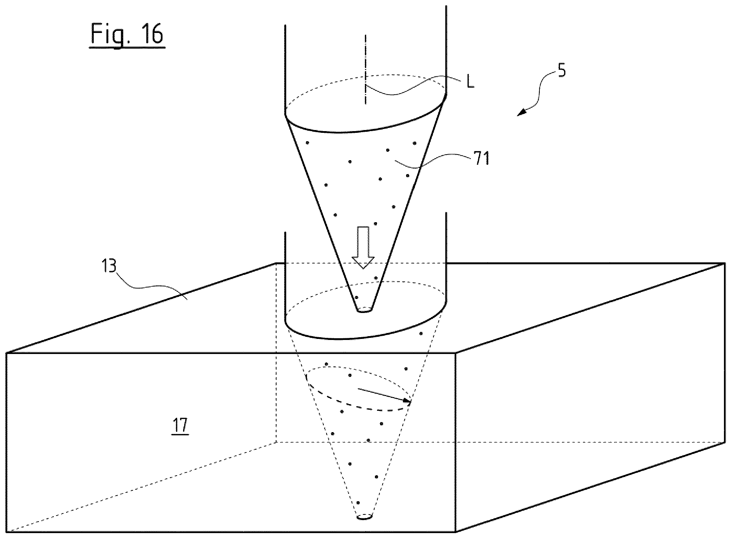

In Fig. 16 ist das prinzipielle perkutane (durch die Haut 13) Einstechen eines Lichtapplikators 5 in Gewebe 17 verdeutlicht. Mit der sich distalwärts verjüngenden Nadelspitze wird die durchstochene Haut 13 und das Gewebe 17 so weit aufgeweitet, dass der Lichtapplikators 5 hindurch passt. Je spitzer die Nadelspitze ist, desto behutsamer geschieht diese Aufweitung der Haut 13 und des Gewebes 17 und desto geringer ist die Kraft, die der Anwender dazu aufbringen muss.

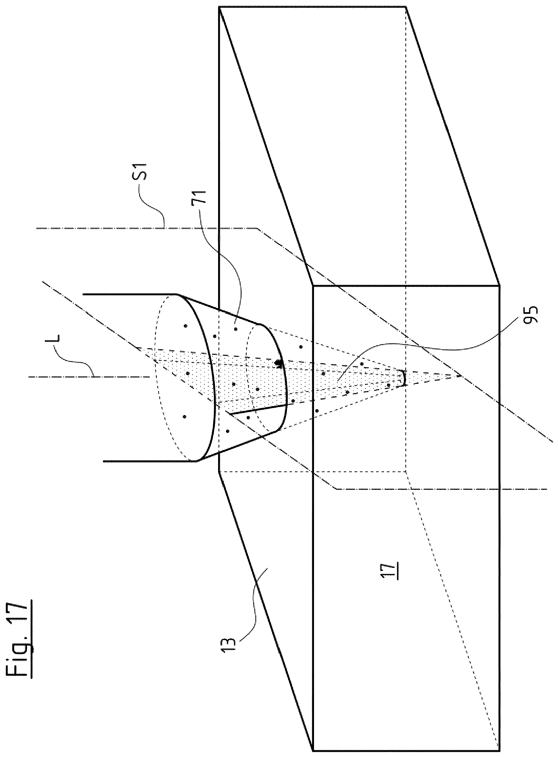

In Fig. 17 ist das Verstärkungselement 95 als dreieckige dünne scharfe Klinge ausgeführt, die in den kegelförmigen Streukörper 71 eingebettet ist und sowohl distal als auch lateral aus dem Streukörper 71 herausragt. Die Klinge 95 definiert eine Längsschnittebene S1, die parallel zur Längsachse L des Lichtapplikators 5 verläuft. Die Klinge 95 durchsticht und zerschneidet beim Einstich des Lichtapplikators 5 das zu passierende Gewebe, um die Gewebeaufweitung zu erleichtern und den Widerstand zu reduzieren. Insbesondere bei festem Gewebe 17 und für das Durchdringen zäher Hautschichten ist dies sinnvoll.

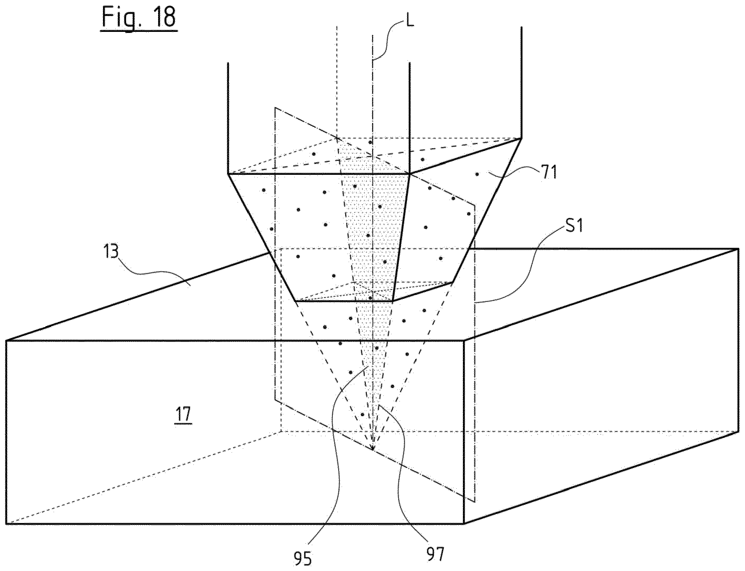

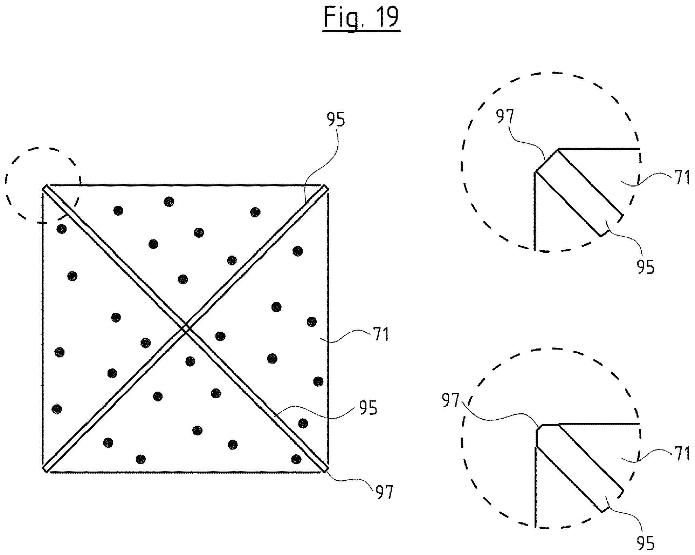

In Fig. 18 ist eine Ausführungsform gezeigt, bei der der Streukörper 71 pyramidenförmig mit im Wesentlichen quadratischer Grundfläche ist. Wie in Figur 19 hat der Einführabschnitt 11 des Lichtapplikators 5 hier einen quadratischen Querschnitt. Das Verstärkungselement 95 ist hier ebenfalls als dreieckige dünne scharfe Klinge ausgeführt, die jedoch vollständig in den kegelförmigen Streukörper 71 eingebettet ist und nicht oder kaum distal oder lateral aus dem Streukörper 71 herausragt. Die Längsschnittebene S1, in der die Klinge 95 liegt, wird hier von der Diagonalen der quadratischen Grundfläche des Streukörpers 71 und der Längsachse L des Lichtapplikators 5 aufgespannt. Die Kanten 97 des Streukörpers 71 bilden hier von der Klinge 95 verstärkte Schnittkanten (siehe Detailansichten rechts in Fig. 19). In Fig. 19 ist das Verstärkungselement als zwei im Querschnitt im rechten Winkel gekreuzt zueinander angeordnete Klingen gezeigt. Es entsteht dadurch beim Einstechen ein Kreuzschnitt, mit dem sich das Gewebe besonders leicht aufweiten lässt.

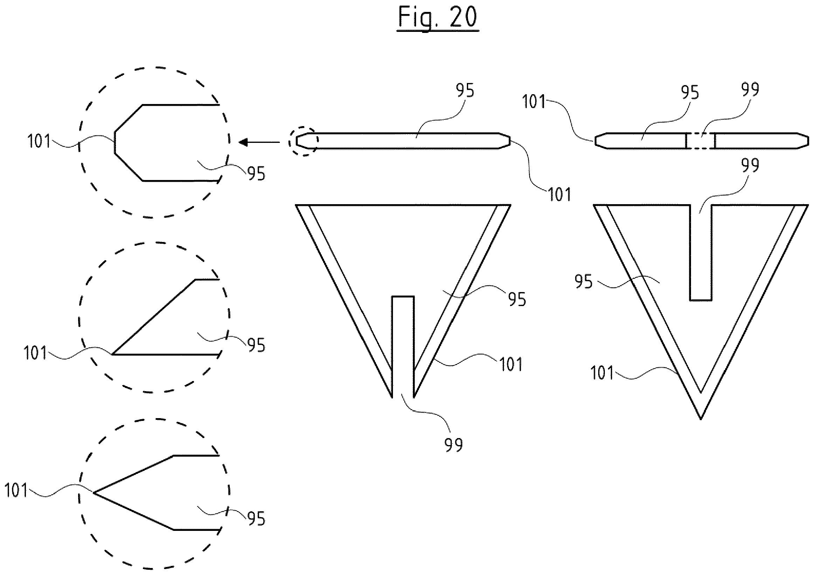

Wie in Fig. 20 gezeigt, können die gekreuzten Klingen 95 auf verschiedene Weise am Schneidrand scharf geschliffen sein. Die beiden Klingen 95 können jeweils korrespondierende Schlitze 99 aufweisen, sodass die Klingen gekreuzt ineinandergesteckt werden können. Die Schlitze 99 erstrecken sich jeweils in Längsrichtung in etwa über die Hälfte der Klingen 95. Die Breite der Schlitze 99 entspricht in etwa der Dicke der Klingen 95.

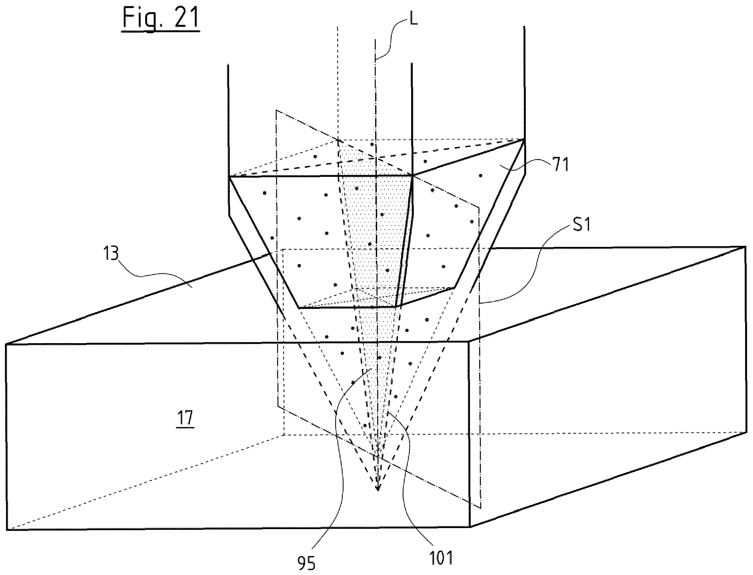

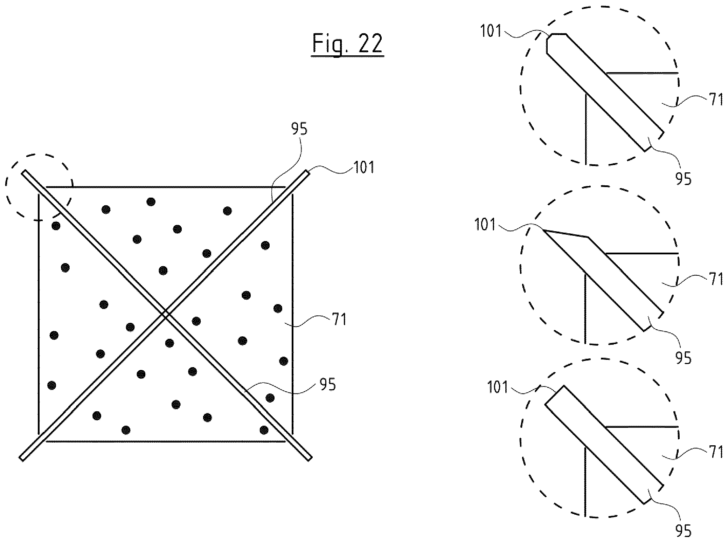

Aus den Figuren 21 und 22 wird deutlich, dass auch bei einem pyramidenförmigen Streukörper 71 mit quadratischer Grundfläche das Verstärkungselement 95 in Form von gekreuzten Klingen 95 zur Bildung von schärferen Schneidkanten 101 über den Streukörper 71 sowohl distal als auch lateral hinausragen kann.

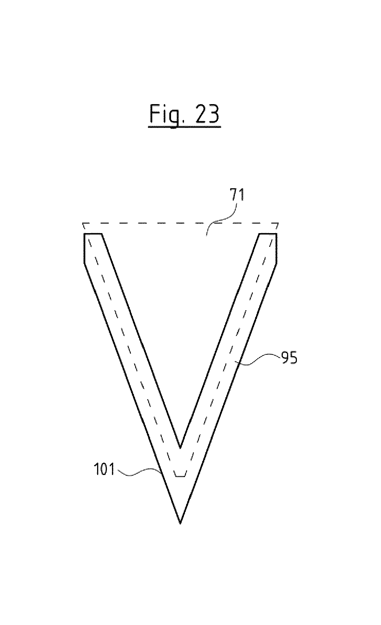

Das in Figur 23 gezeigte Verstärkungselement 95 ist eine V-förmige Klinge, um die von der Klinge 95 im Streukörper 71 gebildete lichtundurchlässige Fläche in der Schneidebene zu verringern. Bei den Klingen gemäß Figuren 17 bis 22 kann die Klingenfläche allerdings gut lichtreflektierend ausgestaltet sein, sodass dies gut kompensiert werden kann.

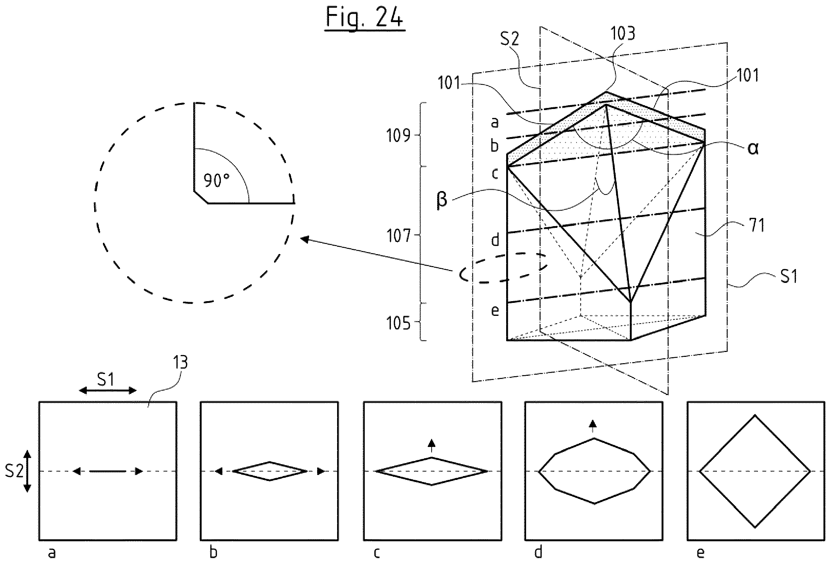

Bei den in Figuren 24 bis 27 gezeigten Ausführungsbeispielen wird bewusst auf eine gekreuzte Konfiguration von zwei Klingen als Verstärkungselement 95 verzichtet und nur eine Klinge als Verstärkungselement 95 eingesetzt. Der Heilungs- und Vernarbungsprozess eines gekreuzten haut- oder Gewebeschnitts kann in manchen Fällen schlechter verlaufen als ein einzelner Längsschnitt. Außerdem ist die Einbettung einer einzelnen Klinge 95 in den Streukörper 71 fertigungstechnisch einfacher.

Der Streukörper 71 hat in den in Figuren 24 bis 27 gezeigten Ausführungsbeispielen eine besondere polyedrische Form mit quadratischer Grundfläche. Der Streukörper 71 läuft in einer ersten Längsschnittebene S1 in einem ersten Winkel $\alpha$ distalwärts spitz zu und in einer zur ersten Längsschnittebene S1 senkrecht liegenden zweiten Längsschnittebene S2 in einem zweiten Winkel $\beta$ distalwärts spitz zu, wobei der zweite Winkel $\beta$ spitzer ist als der erste Winkel $\alpha$. In der ersten Längsschnittebene S1 ist die Klinge 95 als Verstärkungselement eingebettet und ragt distal, aber nicht lateral aus dem Streukörper 71 heraus. Die Schnittkanten 101 der Klinge 95 verlaufen im Winkel $\alpha$ zueinander und bilden eine Spitze 103. In der zweiten Längsschnittebene S2 wird das Gewebe nicht geschnitten, sondern behutsam auseinandergedrückt, und zwar erst dann, wenn der Schnitt in der ersten Längsschnittebene S1 zum Großteil bereits gemacht wurde. Zum behutsamen Auseinanderdrücken des Gewebes in der Längsschnittebene S2 tragen auch die stumpfen Winkel der Kanten des Streukörpers 71 in der Längsschnittebene S2 bei.

In Fig. 24 unten sind die Phasen a-e der Haut- bzw. Gewebeöffnung gezeigt, wenn die Nadelspitze die entsprechend gekennzeichnete Eindringtiefe erreicht hat. Anfänglich in Phase a wird nur in der ersten Längsschnittebene S1 geschnitten und in Phase b nur wenig zusätzlich in der zweiten Längsschnittebene S2 das Gewebe auseinandergedrückt. In Phase c ist der Schnitt in der ersten Längsschnittebene S1 vollendet. Der Großteil der Aufweitung der Haut- bzw. Gewebeöffnung in der zweiten Längsschnittebene S2 findet erst in den Phasen d und e statt, also dann, wenn bereits die Gewebeöffnung durch einen definierten Schnitt erzielt wurde.

Der Streukörper 71 gemäß den in Figuren 24 bis 27 gezeigten Ausführungsbeispielen weist einen ersten proximalen Streukörperabschnitt 105 mit quadratischem Querschnitt auf (siehe Phase e). Distal vom ersten Streukörperabschnitt 105 erstreckt sich ein zweiter Streukörperabschnitt 107 und distal vom zweiten Streukörperabschnitt 107 erstreckt sich ein dritter Streukörperabschnitt 109. Der zweite Streukörperabschnitt 107 hat einen im Wesentlichen achteckigen Querschnitt (siehe Phase d) und der dritte Streukörperabschnitt 109 hat einen im Wesentlichen rautenförmigen Querschnitt (siehe Phasen b und c). Das Verstärkungselement 95 verläuft in der Längsschnittebene S1 entlang der längeren Rautendiagonalen im dritten Streukörperabschnitt 109.

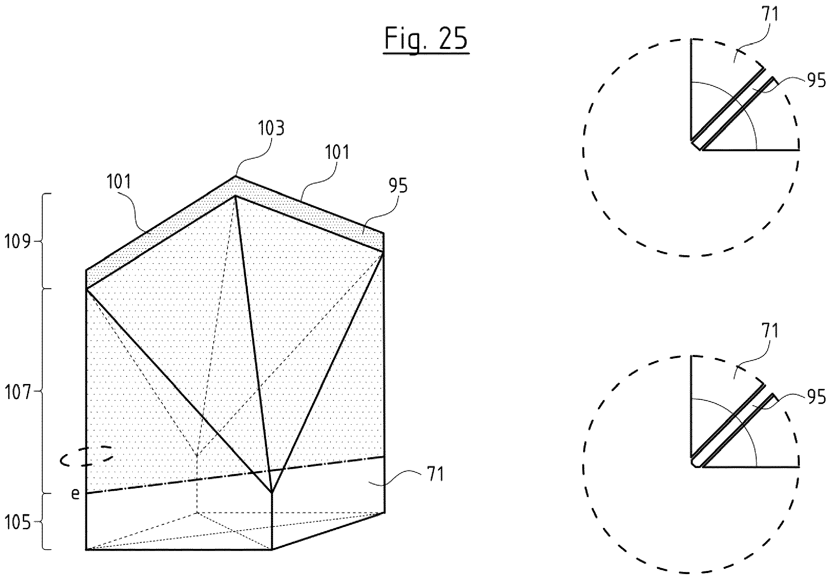

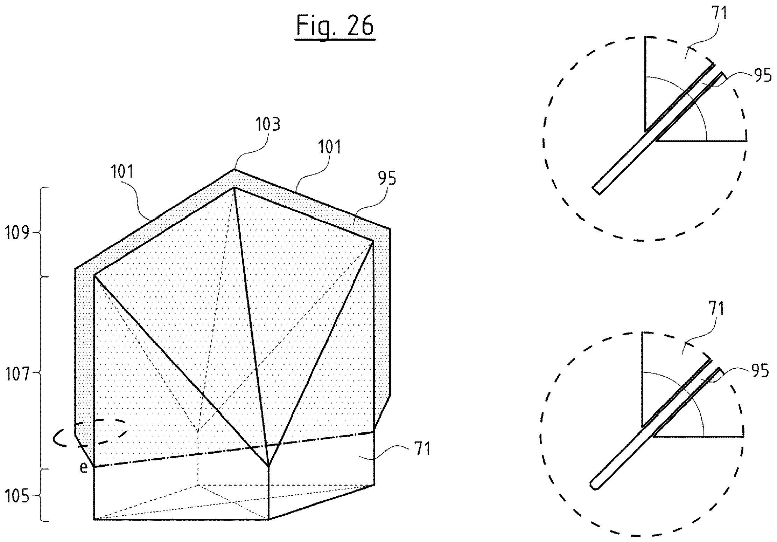

Wie in Fig. 25 und 26 gezeigt, kann sich die Klinge 95 auch über den zweiten Streukörperabschnitt 107 erstrecken und lateral mit dem Streukörper 71 abschließen (Fig. 25) oder lateral herausragen (Fig. 26). Bei festerem Gewebe kann eine größere Klinge 95 (Fig. 26) vorteilhaft sein, bei weicherem Gewebe eine kleinere Klinge 95 (Fig. 25). Die in Fig. 26 gezeigte, lateral über den Streukörper 71 hinausragende Klinge 95 bewirkt einen definierten Gewebeschnitt entlang der Klinge 95. Bevor das Gewebe durch den Prozess des Aufweitens während des Vorschiebens des Applikators an einer undefinierten anderen Stelle aufreißt, soll es gezielt in der Ebene der Klinge 95 aufgeschnitten und damit die Gewebeöffnung vergrößert werden.

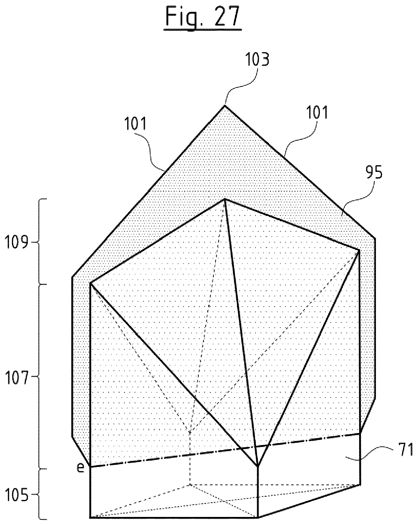

In Fig. 27 ist eine vorteilhafte Ausführungsform gezeigt, bei der die Klinge 95 wie in Fig. 26 lateral herausragt, und dessen Schnittkanten 101 spitzer zueinander laufen als der Winkel $\alpha$ und somit eine spitzere Spitze 103 bilden. Das Verstärkungselement 95 ragt daher mit der Spitze 103 in der radialen Mitte des Streukörpers 71 weiter distalwärts aus dem Streukörper 71 als am radialen Außenbereich des Streukörpers 71. Dies vereinfacht den Einstich in festes Gewebe, wobei u.a. die vom Anwender aufzubringende Kraft reduziert wird.

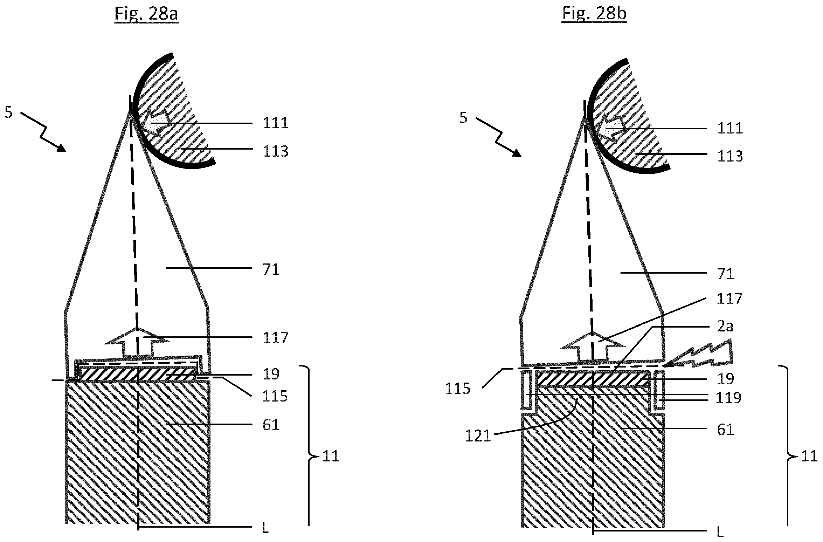

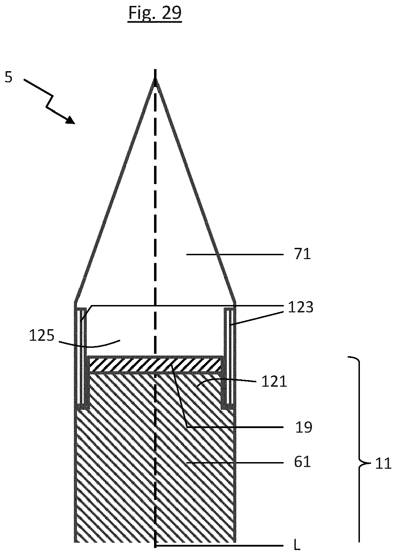

Fig. 28a,b veranschaulichen eine Problematik, die mit dem zuvor beschriebenen Verstärkungselement 95 allein nicht gelöst wird. Während das Verstärkungselement 95 das distal spitz zulaufende Ende der Nadelspitze bzw. des Streukörpers 71 verstärkt und verschärft, können Kräfte 111, die mittels eines externen Körpers 113 seitlich auf die Nadelspitze bzw. den Streukörper 71 einwirken, dazu führen, dass die Nadelspitze bzw. der Streukörper 71 an einer Verbindungsstelle 115 mit dem distalen Ende des Einführabschnitts 11 abbricht oder abreißt. In den in Fig. 28a,b bis Fig. 37a-c gezeigten Ausführungsbeispielen ist die Nadelspitze 71 besonders spitz und im Vergleich zur lateralen Ausdehnung relativ lang ausgeführt, vorzugsweise 1,5-mal so lang oder länger. Dadurch können seitlich einwirkende Kräfte 111 ein relativ hohes Verkippungsmoment auf die Nadelspitze 71 mit resultierender Zugkraft 117 in distale Richtung ausüben, welcher die Verbindungsstelle 115 standhalten muss. In Fig. 28a ist bespielhaft gezeigt, wie sich ggf. die Nadelspitze 71 durch die Zugkraft 117 an der Verbindungsstelle 115 lösen kann. An der Verbindungsstelle 115 kann die Nadelspitze 71 beispielsweise mit der LED 19 und direkt oder indirekt mit dem Leiter 61 verklebt sein. Das Risiko eines solchen Abrisses wird durch die in den Fig. 29 bis Fig. 31 gezeigten Ausführungsbeispiele erheblich reduziert. In Fig. 28b ist ein anderer Fall eines Abbruchs gezeigt, bei dem nicht nur die Verklebung an der Verbindungsstelle 115 versagt, sondern auch das Material der Nadelspitze 71 bricht. Es kann nämlich für die Verklebung und den Zusammenbau von Vorteil sein, insbesondere dann, wenn die Nadelspitze 71 einen proximalseitigen Hülsenfortsatz 119 aufweist, der die LED 19 umfangseitig umgreift und einen gewissen Formschluss mit dem Leiter 61 bildet. Der Leiter 61 kann einen entsprechend verjüngten distalen Endabschnitt 121 zur Aufnahme des Hülsenfortsatzes 119 aufweisen. Allerdings ist der Hülsenfortsatz 119 mit einer radialen Dicke von 300 Mikrometern oder weniger außerordentlich dünn und entsprechend bruchempfindlich. Fig. 28b zeigt einen Abbruch des Hülsenfortsatzes 119 durch die seitlich einwirkende Kraft 111. Ein Abriss (Fig. 28a) oder ein Abbruch (Fig. 28b) der Nadelspitze 71 im Körper des Patienten kann dazu führen, dass die Nadelspitze 71 und/oder Bruchstücke davon operativ aus dem Körper des Patienten entfernt werden müssen. Es gilt, dieses Risiko zu verringern.

Fig. 29 zeigt zur Lösung dieses Problems ein umfangseitig das distale Ende des Einführabschnitts 11 und die Nadelspitze 71 zumindest teilweise umgebendes Stabilisierungselement 123, das sowohl mit dem Einführabschnitt 11 als auch mit der Nadelspitze 71 verbunden ist. Hier ist das Stabilisierungselement 123 direkt radial außenseitig mit dem distalen Endabschnitt 121 des Leiters 61 und einem proximalen Nadelspitzenabschnitt 125 verklebt. Die Verbindung kann jeweils, wie hier gezeigt, durch einen Stoffschluss, wie etwa eine Verklebung, bewirkt werden. Alternativ oder zusätzlich kann ein Kraftschluss, beispielsweise in Form eines Reibschlusses, und/oder ein Formschluss jeweils die Verbindungen bewirken oder unterstützen. Das Stabilisierungselement 123 weist ein Material auf, das zumindest in Längsrichtung L axial verstreckt ist. Der distale Endabschnitt 121 des Leiters 61 und der proximale Nadelspitzenabschnitt 125 sind so weit verjüngt, dass das Stabilisierungselement 123 vollständig radial eingelassen ist und den Einführabschnitt 11 nicht lateral verbreitert.

Das verstreckte Material des Stabilisierungselements 123 kann hier monoaxial, also vornehmlich in die Längsrichtung L, oder bi-axial, d.h. zusätzlich in Umfangsrichtung, verstreckt sein. Hier ist das verstreckte Material ein Kunststoff in Form einer Folie, wobei der Kunststoff eine gegenüber dem unverstreckten Kunststoff um ein Vielfaches erhöhte Zugfestigkeit in der Längsrichtung L aufweist. Die Molekülketten des Stabilisierungselements 123 weisen eine Vorzugsausrichtung in die Längsrichtung L auf, sind in diese Vorzugsrichtung gestreckt, liegen dadurch in einer Ebene quer zur Vorzugsrichtung enger bzw. dichter nebeneinander und bewirken dadurch die Erhöhung der Zugfestigkeit in die Längsrichtung L. In diesem Beispiel weist das Stabilisierungselement 123 axial in Längsrichtung L eine vielfach höhere Zugfestigkeit auf als in der zur Längsrichtung orthogonalen Umfangsrichtung U. Das verstreckte Material des Stabilisierungselement 123 ist dabei monoaxial in Längsrichtung L verstreckt. Durch das verstreckte Material kann das Stabilisierungselement besonders dünn und zugleich besonders zugfest in der Längsrichtung L ausgestaltet sein. Damit wird das Risiko verringert, dass zum einen die Nadelspitze 71 in dem proximalen Nadelspitzenabschnitt 125 oder an dem proximalseitigen Hülsenfortsatz 119 abbricht und zum anderen die Nadelspitze 71 als Ganzes vom distalen Ende des Einführabschnitts 11 abreißt. Wirkt die Kraft 111 quer zur Längsrichtung L auf die Nadelspitze 71 ein, so kann das radial außen angeordnete und in Längsrichtung L besonders zugfeste Stabilisierungselement 123 die resultierende Zugkraft 117 aufnehmen und einen Abbruch oder Abriss der Nadelspitze 71 verhindern.

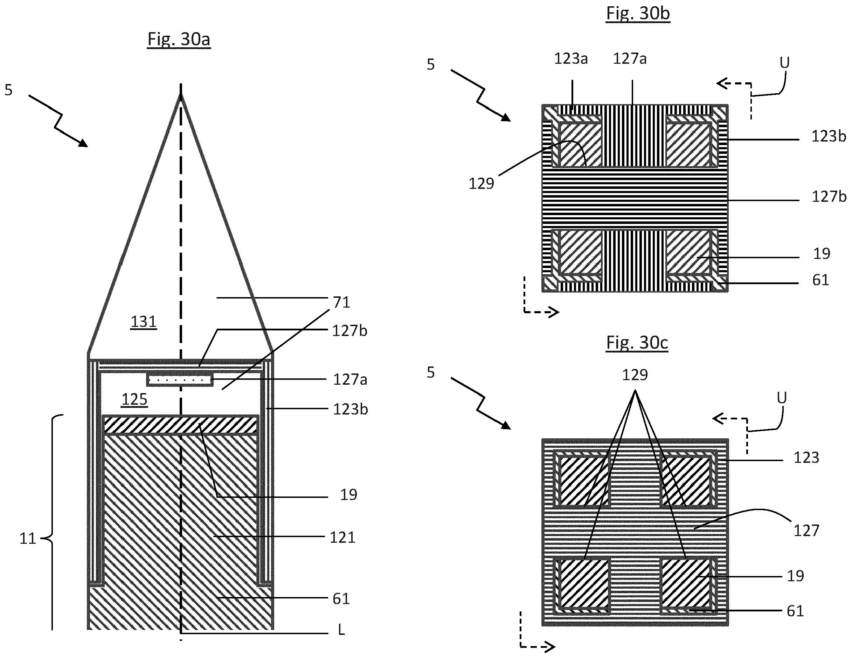

Fig. 30a,b zeigen eine Ausführungsform eines mehrteiligen, hier zweiteiligen, Stabilisierungselements 123a,b in Form von überkreuzten Streifen, die einen Formschluss mit der Nadelspitze 71 bilden, indem jeweils ein radial verlaufender distaler Stabilisierungselementabschnitt 127a,b zumindest teilweise in die Nadelspitze 71 eingegossen bzw. von dieser umgossen ist. Der proximale Nadelspitzenabschnitt 125 wird dabei von jeweils einem der Stabilisierungselemente 123a,b auf gegenüberliegenden lateralen Seiten eingefasst. Die radial verlaufenden distalen Stabilisierungselementabschnitte 127a,b sind schmaler ausgestaltet als der Rest der Stabilisierungselemente 123a,b, sodass in der gekreuzten Anordnung in Fig. 30b Durchbrechungen 129 gebildet werden, durch welche ein distaler Nadelspitzenabschnitt 131 mit dem proximalen Nadelspitzenabschnitt 125 stoffschlüssig, hier integral als eine verschmolzene Vergussmasse, verbunden ist. Hier ist die Querschnittsform des Lichtapplikators 5 im Wesentlichen quadratisch, sodass die vier lateralen Seiten des proximalen Nadelspitzenabschnitts 125 im Wesentlichen vollflächig eingefasst sind. Die Querschnittsform des Lichtapplikators 5 kann allerdings optional kreisrund, oval, rechteckig, sechseckig oder allgemein polygonal sein.

Fig. 30c zeigt eine Ausführungsform eines einteiligen, strumpfförmigen Stabilisierungselements 123, das einen Formschluss mit der Nadelspitze 71 bildet, indem ein radial verlaufender distaler Stabilisierungselementabschnitt 127 zumindest teilweise in die Nadelspitze 71 eingegossen bzw. von dieser umgossen ist. Das Stabilisierungselement 123 weist dabei im distalen Stabilisierungselementabschnitt 127 axiale Durchbrechungen 129 auf, durch welche der distale Nadelspitzenabschnitt 131 mit dem proximalen Nadelspitzenabschnitt 125 stoffschlüssig, hier integral als eine verschmolzene Vergussmasse, verbunden ist.

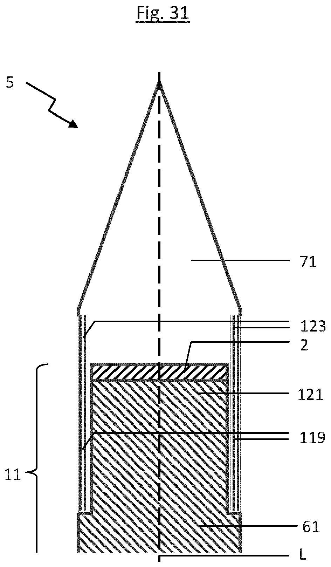

Im Ausführungsbeispiel gemäß Fig. 31 ist das Stabilisierungselement 123 im proximalseitigen Nadelspitzenhülsenfortsatz 119 integriert, wobei der Nadelspitzenhülsenfortsatz 119 einen Faser-Kunststoff-Verbund mit axial verstreckten Fasern aufweist, wobei die Fasern eine Vorzugsausrichtung in der Längsrichtung L aufweisen. Der Volumenanteil an verstreckten Fasern beträgt hierbei 50% oder mehr. Die radiale Dicke des Nadelspitzenhülsenfortsatzes 119 kann beispielsweise im Bereich von 50 Mikrometer bis 300 Mikrometer liegen. Die axiale Länge des Nadelspitzenhülsenfortsatzes 119 kann, wie hier gezeigt, weit in proximale Richtung des Einführabschnitts 11 reichen, beispielsweise den Durchmesser des Einführabschnitts 11 übersteigen, um eine möglichst große radiale Fläche für einen Stoffschluss mit dem distalen Ende des Einführabschnitts 11 durch Verkleben zu bereitzustellen. Das Stabilisierungselement 123 ist über mehr als 50% seiner axialen Länge, vorzugsweise aber über seine gesamte axiale Länge, radial innenseitig verklebt. Optional kann das Stabilisierungselement 123 zusammen mit der Nadelspitze 71 als Guss- oder Spritzgussteil ausgeführt sein und dementsprechend durch Vergießen oder Umspritzen mit dem distalen Endabschnitt 121 des Leiters 61 verbunden sein.

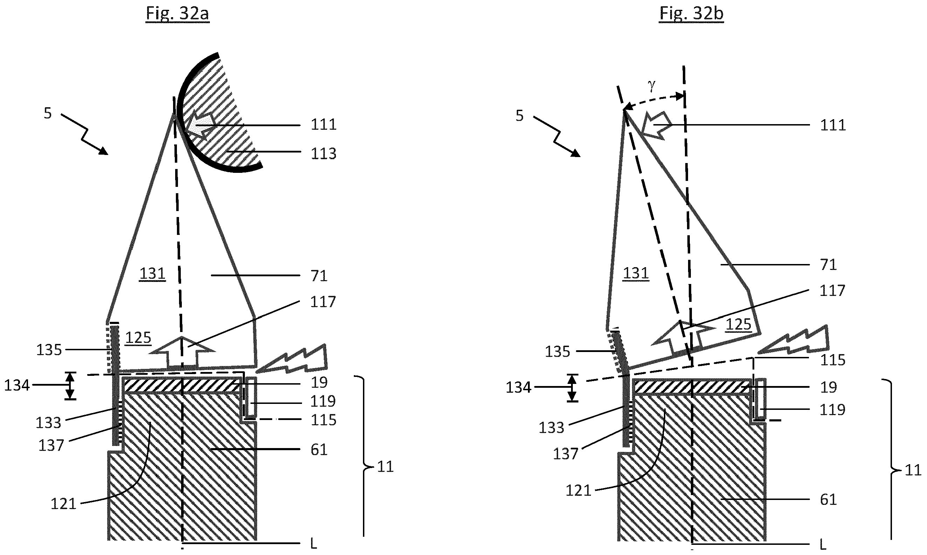

In Fig. 32a,b wird ein weiterer Aspekt der vorliegenden Offenbarung verdeutlicht, der für sich allein genommen oder in Kombination mit den zuvor beschriebenen Aspekten Anwendung finden kann. Es besteht das Problem, dass selbst bei Einsatz eines oben beschriebenen Stabilisierungselements 123, und erst recht ohne ein Stabilisierungselement 123, potentiell immer eine Kraft 111 seitlich einwirken kann, die groß genug ist, um die Nadelspitze 71 abzureißen bzw. abzubrechen. Um in einem solchen Fall den vollständigen Abriss und einen Verbleib der Nadelspitze 71 oder Teilen davon im Körper des Patienten zu verhindern, ist hier ein zumindest teilweise umfangseitig das distale Ende des Einführabschnitts 11 und zumindest teilweise umfangseitig die Nadelspitze 71 umgebendes Sicherheitselement 133 mit dem Einführabschnitt 11 und der Nadelspitze 71 verbunden. Im Gegensatz zum Stabilisierungselement 123 trägt das Sicherheitselement 133 nicht oder nur marginal zur Erhöhung der axialen Zugfestigkeit bei. Das Sicherheitselement 133 ist zumindest über bestimmte bzw. definierte axiale Bereiche hinweg flexibel und sichert die Nadelspitze 71 im Falle eines Bruchs oder Abrisses des Stabilisierungselements 123 und/oder der Nadelspitze 71 am distalen Ende des Einführabschnitts 11 vor einem vollständigen Abreißen vom Lichtapplikator 5. Bei dem Sicherheitselement 133 geht es also nicht um eine Erhöhung der Stabilität, sondern um eine Sicherheitsmaßnahme für den Fall, dass die auf die Nadelspitze 71 einwirkenden Kräfte 111, 117 so groß sind, dass die Nadelspitze 71 abreißt, auseinanderreißt oder bricht. Das Sicherheitselement 133 ist deshalb besonders vorteilhaft, weil es im Gegensatz zum Stabilisierungselement 123 eine Flexibilität hat, die das Stabilisierungselement 123 von vornherein nicht hat oder beim Stabilisierungselement 123 durch das Verstrecken reduziert ist oder zumindest nicht verfügbar ist bzw. nicht genutzt werden kann, weil das Stabilisierungselement 123 vorzugsweise über seine gesamte axiale Länge mit dem Lichtapplikator 5 fest, z.B. stoffschlüssig, verbunden ist. Das Sicherheitselement 133 unterscheidet sich hier vom Stabilisierungselement 123 des Weiteren dadurch, dass es zwischen seiner festen Verbindung mit dem distalen Ende des Einführabschnitts 11, die stoff-, kraft-/reib- oder formschlüssig ausgeführt sein kann, einerseits, und seiner festen Verbindung mit der Nadelspitze 71, die gleichfalls stoff-, kraft-/reib- oder formschlüssig ausgeführt sein kann, andererseits, einen axialen Abstand 134 gibt, der die Verbindungsstelle 115 zwischen dem distalen Ende des Einführabschnitts 11 und der Nadelspitze 71 umgibt bzw. einschließt. Dieser axiale Abstand 134 ist dadurch definiert, dass das Sicherheitselement 133 dort weder mit dem distalen Ende des Einführabschnitts 11 noch mit der Nadelspitze 71 fest verbunden ist, sondern gegenüber Einführabschnitt 11 und Nadelspitze 71 beweglich, verschiebbar und/oder biegbar bleibt oder zumindest spätestens mit dem Bruch oder Abriss der Nadelspitze 71 beweglich, verschiebbar und/oder biegbar wird. Die Flexibilität einerseits und der axiale Abstand 134 zwischen den beiden festen Verbindungen und über die Verbindungsstelle 115 hinweg sind hier die Merkmale des Sicherheitselements 133, die hier das Sicherheitselement 133 vom Stabilisierungselement 123 unterscheiden.

In Fig. 32a ist das Sicherheitselement 133 ein flexibler Metalldraht, der mit einem distalen Sicherheitselementabschnitt 135 mit der Nadelspitze 71 stoffschlüssig verbunden ist, d.h. in diesem Fall verklebt ist. Ein proximaler Sicherheitselementabschnitt 137 ist mit dem distalen Ende des Leiters 61 stoffschlüssig verbunden, d.h. in diesem Fall verlötet. Zwischen diesen beiden Verbindungen befindet sich der Abstand 134, der nicht stoffschlüssig verbunden ist, über die Verbindungsstelle 115 hinwegreicht und aufgrund seiner Flexibilität nachgiebig biegsam ist. Im Falle eines wie in Fig. 32a gezeigten Bruches bzw. Abrisses an der Verbindungsstelle 115 zwischen der Nadelspitze 71 und dem distalen Ende des Einführabschnitts 11, kann das Sicherheitselement 133 bei andauernder seitlicher Krafteinwirkung 111 über einen sehr großen Bereich eines Verbiegungswinkels γ resilient nachgeben, d.h. $0 \leq \gamma \leq 180°$. Wie in Fig. 32b gezeigt, führt eine weitere Krafteinwirkung 111 daher nicht zu einem vollständigen Abriss oder Abbruch der Nadelspitze 71 vom Lichtapplikator 5, sondern nur zu einer reversiblen Verbiegung des flexiblen Sicherheitselements 133. Die zerstörungsfreie weitere Verbiegung kann dabei plastisch und/oder elastisch erfolgen. Beispielsweise kann ein relativ weicher Metalldraht, etwa aus Kupfer, vornehmlich plastisch nachgeben, wohingegen ein relativ harter Metalldraht, etwa aus Federstahl, vornehmlich elastisch nachgeben kann. Optional kann der flexible Metalldraht 133 zunächst über seine gesamte Länge mit der Nadelspitze 71 und dem distalen Ende des Einführabschnitts 11 stoffschlüssig fest verbunden sein. Bedingt durch einen Abriss oder Abbruch der Nadelspitze 71 kann allerdings dann der Abstand 134 entstehen, der es dem Metalldraht 133 ermöglicht, seine kennzeichnende Flexibilität zu nutzen und sich über den Abstand 134 elastisch oder plastisch zu verbiegen.

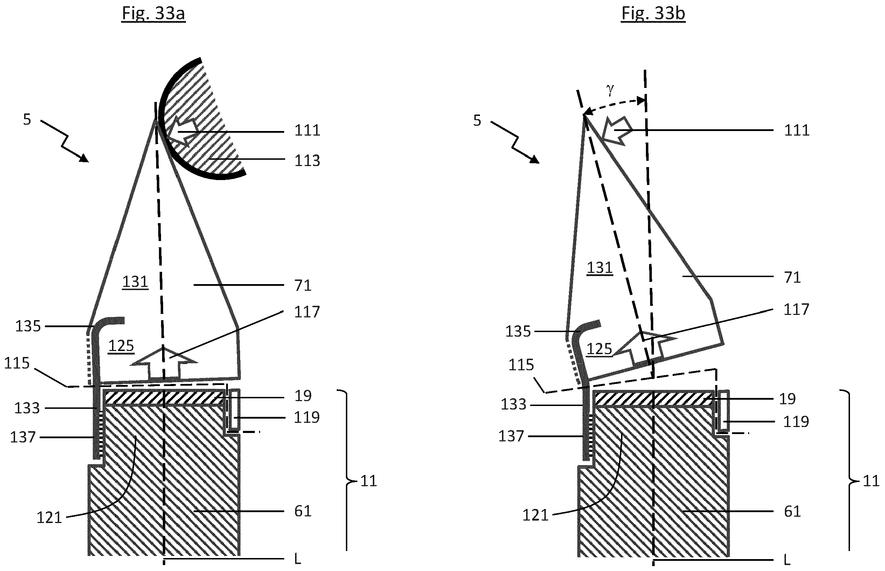

Die Fig. 33a,b unterscheiden sich von den Fig. 32a,b dadurch, dass der distale Sicherheitselementabschnitt 135 nicht nur mit der Nadelspitze 71 verklebt ist, sondern sich zumindest teilweise radial in die Nadelspitze 71 hinein erstreckt und somit einen Formschluss mit der Nadelspitze 71 bildet. Dies kann beispielsweise dadurch erreicht werden, dass die Nadelspitze 71 als Guss- oder Spritzgussteil ausgeführt ist und der distale Sicherheitselementabschnitt 135 vergossen oder umspritzt ist. Dadurch ist die Nadelspitze 71 noch besser durch das Sicherheitselement 133 gesichert.

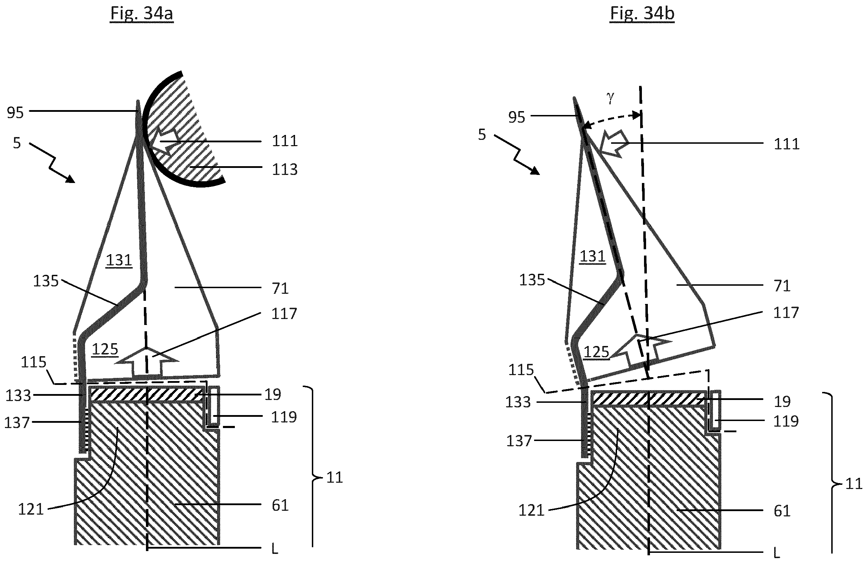

In Fig. 34a,b ist das Sicherheitselement 133 so gebogen bzw. gewinkelt geformt und erstreckt sich mit dem distalen Sicherheitselementabschnitt 135 radial mittig, wobei der distale Sicherheitselementabschnitt 135 ein radial mittiges Verstärkungselement 95 in Form einer Metallspitze der Nadelspitze 71 bildet. Damit kann der distale Sicherheitselementabschnitt 135 das distale Ende der Nadelspitze 71 verstärken und/oder als distaler Nadelspitzenfortsatz fungieren.

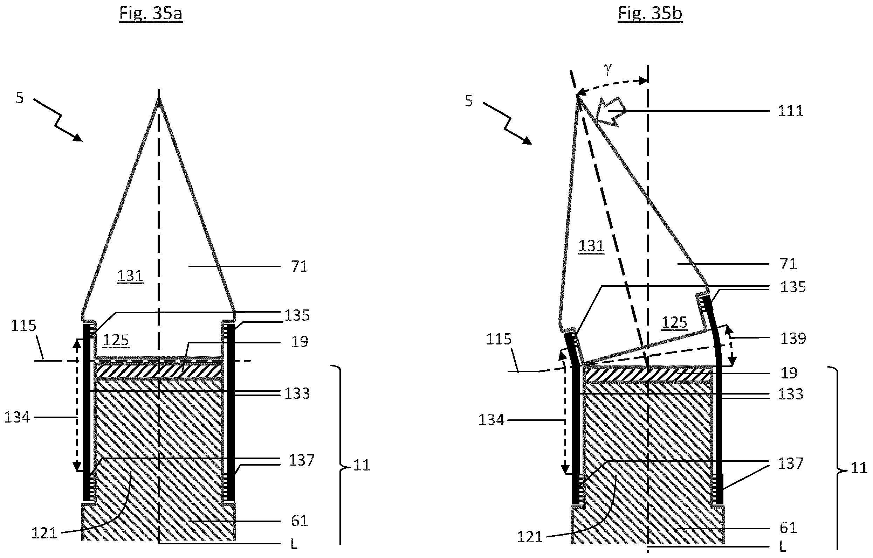

In Fig. 35a,b umgreift das Sicherheitselement 133 schlauchförmig vollständig umlaufend das distale Ende des Einführabschnitts 11 und den proximalen Nadelspitzenabschnitt 125 sowie die axiale Verbindungsstelle 115 zwischen diesen. Das hat den Vorteil, dass bei Abbruch und/oder Abriss der Nadelspitze 71 etwaige Bruchstücke oder Splitter innerhalb des Sicherheitselements 133 verbleiben und nicht in den Körper des Patienten eindringen. Besonders im Bereich der Verbindungsstelle 115 mit einer stoffschlüssigen Klebeverbindung zwischen Nadelspitze 71 und dem distalen Ende des Einführabschnitts 11 kann das Risiko für einen Bruch oder Abriss erhöht sein, sodass dort abbrechende Bruchstücke oder Splitter vom Sicherheitselement 133 umschlossen bleiben und nicht in den Körper des Patienten eindringen. Beispielsweise kann das Sicherheitselement 133 ein Schrumpfschlauch sein, der zumindest teilweise außenseitig auf das distale Ende des Einführabschnitts 11 und den proximalen Nadelspitzenabschnitt 125 thermisch aufgeschrumpft ist. Vorzugsweise ist das Sicherheitselement 133 in dieser Ausführungsform lichttransparent, beispielsweise als lichttransparenter Kunststoffschlauch ausgeführt. Das Sicherheitselement 133 unterscheidet sich auch hier vom Stabilisierungselement 123 im Wesentlichen dadurch, dass es erstens zumindest über bestimmte bzw. definierte axiale Bereiche hinweg flexibel ist und zweitens zwischen seiner ersten Verbindung mit dem distalen Ende des Einführabschnitts 11 und der zweiten Verbindung mit der Nadelspitze 71 den axialen Abstand 134 aufweist, über welchen das hier als Schrumpfschlauch ausgeführte Sicherheitselement 133 beweglich bzw. verschiebbar an der Nadelspitze 71 und/oder am distalen Ende des Einführabschnitts 11 anliegt. Lediglich distalseitig bzw. proximalseitig vom axialen Abstand 134 ist das Sicherheitselement 133 über den jeweiligen Sicherheitselementabschnitt 135 bzw. 137 fest, im Sinne von unbeweglich bzw. nicht verschiebbar, mit der Nadelspitze 71 bzw. mit dem distalen Ende des Einführabschnitt 11 verbunden, z.B. reib-/kraftschlüssig thermisch angeschrumpft und/oder stoffschlüssig verklebt. Wie in Fig. 35b gezeigt, weist das Sicherheitselement 133 eine gewisse maximale Dehnlänge 139 auf, damit die Nadelspitze 71 bei seitlicher Krafteinwirkung 111 über einen relativ großen Bereich eines Verbiegungswinkels $\gamma$ resilient nachgeben kann, d.h. $\gamma \geq 20°$. Die maximale Dehnlänge 139 und der maximale Verbiegungswinkel $\gamma$, welche die resiliente Nachgiebigkeit definieren, werden von zwei Größen bestimmt, nämlich von der Dehnbarkeit des Sicherheitselements 133 im Bereich des axialen Abstands 134 und von der Länge des axialen Abstands 134 selbst. Bei der Dehnbarkeit handelt es sich um eine Materialkonstante, die angibt, um welche Länge ein Werkstoff gegenüber seiner ursprünglichen Länge gedehnt werden kann, bevor er bricht oder reißt. Hat also beispielsweise das Material des Sicherheitselements 133 nur eine relativ geringe Dehnbarkeit, dann kann zum Ausgleich dafür der axiale Abstand 134 entsprechend länger ausgeführt werden, um eine genügend große maximale Dehnlänge 139 und damit einen maximalen Verbiegungswinkel $\gamma$ mit resilienter Nachgiebigkeit erzielen zu können. Andersherum kann der axiale Abstand 134 kürzer gewählt werden, wenn das Material des Sicherheitselements 133 eine relativ hohe Dehnbarkeit aufweist.

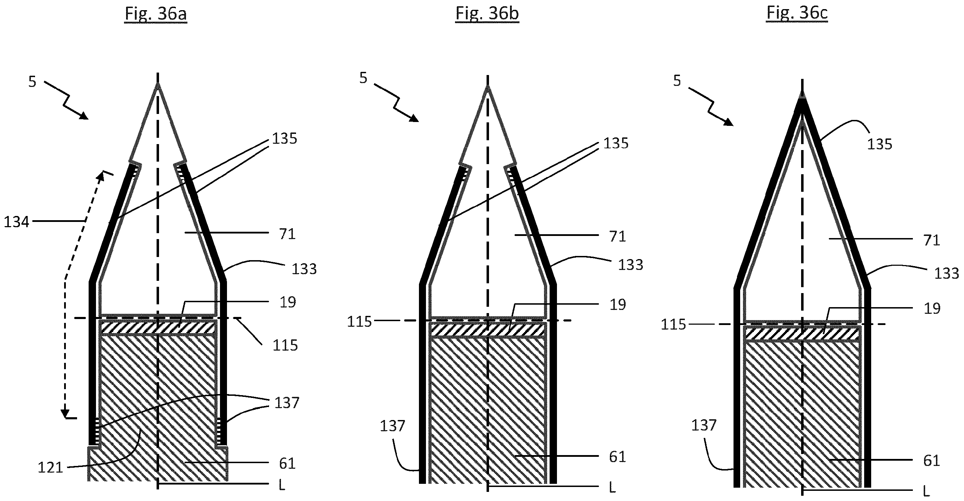

Der distale Sicherheitselementabschnitt 135 des schlauchförmigen Sicherheitselements 133, z.B. in Form eines lichttransparenten Schrumpfschlauchs, kann korrespondierend mit der sich distalwärts verjüngenden Nadelspitze 71 außenseitig an der Nadelspitze 71 radial zulaufen, wie in Fig. 36a-c gezeigt. Dadurch kann ein Formschluss erzielt werden, der das Risiko eines Abrisses verringert. Es ist dabei vorteilhaft, wenn der distale Sicherheitselementabschnitt 135 Material aufweist, das in Umlaufrichtung U verstreckt ist. Dadurch dehnt sich der distale Sicherheitselementabschnitt 135 weniger leicht radial auf und bildet einen entsprechend stärkeren Formschluss mit der Nadelspitze 71. Alternativ oder zusätzlich kann der Formschluss des distalen Sicherheitselementabschnitts 135 dadurch verstärkt werden, dass am schlauchförmigen Sicherheitselement 133 eine axiale Verstreckung oder, sofern das schlauchförmige Sicherheitselement 133 bereits geringfügig bzw. moderat verstrecktes Ausgangsmaterial aufweist, eine zusätzliche axiale Verstreckung bzw. eine axiale Nachverstreckung mit thermischer Fixierung vorgenommen wird. Dies steht der Flexibilität des Sicherheitselements 133 zwischen seiner ersten Verbindung mit dem distalen Ende des Einführabschnitts 11 und seiner zweiten Verbindung mit der Nadelspitze 71 nicht entgegen, da eine solche optionale Verstreckung des Sicherheitselements 133 nur lokal bzw. orts-/abschnittsbezogen, d.h. über einen definierten axialen Teilabschnitt durchgeführt wird und wirksam ist, beispielsweise über den sich verjüngenden distalen Sicherheitselementabschnitt 135. Das Sicherheitselement 133 weist dabei auch mindestens einen flexiblen axialen Teilabschnitt auf, der keine axiale Verstreckung oder, im Falle einer bereits vorliegenden geringfügigen oder moderaten Verstreckung, keine zusätzliche axiale Verstreckung bzw. keine axiale Nachverstreckung erfährt, beispielsweise der Bereich um die Verbindungsstelle 115.

Die Gründe und Vorteile der kennzeichnenden Ortsbegrenztheit der durchgeführten axialen Verstreckung oder Nachverstreckung des Sicherheitselements 133 sind folgende. Generell bewirkt eine axiale Verstreckung in dem Bereich, wo sie durchgeführt wird und wirksam ist, eine Erhöhung des Kristallinitätsgrades und ein verstärkte Orientierung der Polymere des schlauchförmigen Sicherheitselements 133 im Vergleich zu den anderen Bereichen, was bedeutet, dass die Molekülketten gestreckt werden, zueinander ausgerichtet werden und vor allen Dingen enger aneinanderrücken und so zwischen den Molekülketten eine physikalische Verbindung entsteht. Hinsichtlich der daraus resultierenden Material- bzw. Werkstoffeigenschaften bedeutet dies wiederum eine Reduktion der Flexibilität (z.B. können bereits gestreckte Molekülketten kaum mehr oder überhaupt nicht mehr weiter gestreckt werden, was schließlich die Dehnbarkeit des Sicherheitselements 133 als Ganzes in entsprechender Weise beeinträchtigt) und eine Zunahme der Dichte, Festigkeit, Steifigkeit, und, daraus resultierend, der Formstabilität im verstreckten Bereich.

In dem sich verjüngenden distalen Sicherheitselementabschnitt 135 ist eine Zunahme der Steifigkeit und der Formstabilität durchaus erwünscht und von Vorteil, da dies, alternativ oder zusätzlich zur oben bereits erwähnten optionalen Verstreckung des schlauchförmigen Sicherheitselements 133 in Umlaufrichtung U dazu führt, dass sich der distale Sicherheitselementabschnitt 135 bei Belastung weniger leicht radial aufdehnen kann und so einen entsprechend stärkeren Formschluss mit der Nadelspitze 71 bildet. Im axialen Teilabschnitt um die Verbindungsstelle 115 hingegen wäre eine Zunahme der Steifigkeit mit weniger Flexibilität und Nachgiebigkeit verbunden und daher von Nachteil.

Die Verjüngung der Nadelspitze 71 in distale Richtung kann in vorteilhafter Weise für eine zusätzliche Erhöhung der Steifigkeit und Formstabilität beim Verstrecken genutzt werden. Mit abnehmendem Durchmesser der Nadelspitze 71 gelangen die durch das axiale Verstrecken axial orientierten und gestreckten Molekülketten des an die Nadelspitzenform adaptierten schlauchförmigen Sicherheitselements 133 immer näher zusammen, so dass die Dichte und damit auch die Steifigkeit und Formstabilität zum distalen Ende der Nadelspitze 71 hin immer weiter zunehmen. Ein belastungsbedingtes Aufdehnen des axial verstreckten schlauchförmigen Sicherheitselements 133 und in der Folge ein Ausbrechen einer durch Belastung abgebrochenen Nadelspitze 71 in axiale bzw. distale Richtung ist damit äußerst unwahrscheinlich. Das Sicherheitselement 133 kann nämlich an der engsten Stelle am distalen Ende die höchste Steifigkeit und Formstabilität haben.

Vorzugsweise wird eine solche lokal begrenzte bzw. auf einen definierten axialen Bereich eingegrenzte axiale Verstreckung oder, falls bereits ein geringfügig bzw. moderat verstrecktes Ausgangsmaterial vorliegt, eine solche lokal eingegrenzte zusätzliche axiale Verstreckung bzw. lokal eingegrenzte axiale Nachverstreckung direkt und unmittelbar am Lichtapplikator 5 selbst durchgeführt. Alternativ dazu kann dies an einer formgleichen Aufbauhilfe (nicht abgebildet) durchgeführt werden, von welcher das schlauchförmige Sicherheitselement 133 nach dem axialen Verstrecken oder zusätzlichen Verstrecken bzw. Nachverstrecken in axiale Richtung wieder abgenommen wird, um es danach dem Lichtapplikator 5 überzustülpen und in seine finale Position zu bringen. Diese Vorgehensweise direkt am bzw. auf dem Lichtapplikator 5, wo das Sicherheitselement 133 final platziert werden soll (oder zumindest an oder auf einer formgleichen Aufbauhilfe) ist besonders vorteilhaft, wenn das schlauchförmige Sicherheitselement 133 im vorgesehenen Bereich (hier beispielweise im konischen Bereich) vorzugsweise derart stark verstreckt oder stark zusätzlich verstreckt bzw. stark nachverstreckt wird, dass es dort seine Flexibilität weitestgehend verliert und stattdessen maximale Dichte und Steifigkeit und daraus resultierend maximale Formstabilität gewinnt. Bei einem Verstrecken auf einem anders geformten Objekt oder gar formlos wäre eine nachträgliche Adaption auf die Form der Nadelspitze 71 aufgrund der mit dem Verstreckungsprozess gezielt angestrebten Steifigkeit und Formstabilität erheblich erschwert. Das bedeutet also, dass für das Sicherheitselement 133 kein bereits final verstreckter Kunststoff verwendet werden kann, wie z.B. beim Stabilisierungselement 123, sondern das finale Verstrecken vorzugsweise anwendungsspezifisch durchgeführt wird.

Die erste Verbindung des Sicherheitselementabschnitts 135 mit der Nadelspitze 71 ist also in der Ausführung der Fig. 36a als Formschluss ausgeführt. Optional kann der Sicherheitselementabschnitt 135 an seinem distalen Ende mit der Nadelspitze zusätzlich stoffschlüssig verbunden, z.B. verklebt sein. Um zu verhindern, dass bei einer Krafteinwirkung 111 bzw. 117 die Nadelspitze 71 mitsamt dem Sicherheitselement 133 vollständig vom distalen Ende des Einführabschnitts 11 getrennt wird, ist der Sicherheitselementabschnitt 137 mit dem elektrischen Leiter 61 beispielhaft als reiner Stoffschluss, z.B. als Verklebung, ausgeführt. Der als freie Länge des Schrumpfschlauchs 133 definierte axiale Abstand 134, derwesentlich die Dehnlänge 139 bestimmt und deshalb vorzugsweise groß auszuführen ist, kann sich dadurch über nahezu die gesamte axiale Länge des Sicherheitselements 133 erstrecken.

Optional kann der Lichtapplikator 5 auch proximal von der Verbindungsstelle 115 im Bereich des Sicherheitselementabschnitts 137 verjüngt sein, d.h. einen geringeren Durchmesser aufweisen (nicht gezeigt). Das Sicherheitselement 133 kann im proximalen Sicherheitselementabschnitt 137 über einen solchen verjüngten axialen Bereich, beispielsweise des Leiters 61, axial und/oder umlaufend verstreckt oder nachverstreckt sein. Analog zum distalen Sicherheitselementabschnitt 135 kann dadurch in diesem Bereich örtlich begrenzt eine erhöhte Steifigkeit und daraus resultierend eine erhöhte Formstabilität erzielt werden. Auch hier kann also eine Verstärkung des Formschlusses dadurch erreicht werden, dass das Sicherheitselement 133 lokal begrenzt, nämlich begrenzt auf den verjüngten axialen Bereich des Sicherheitselementabschnitts 137, axial und/oder umlaufend verstreckt wird. Die durch das Verstrecken erreichte erhöhte Steifigkeit und die daraus resultierende erhöhte Formstabilität führen dazu, dass sich das im verjüngten Bereich verstreckte Sicherheitselement 133 auch bei Belastung kaum noch aufdehnen kann. Dies sichert besser gegen ein Abziehen des Sicherheitselements 133 als ein gewöhnliches Anschrumpfen des Schrumpfschlauches 133. Die Verstreckung des Schrumpfschlauches 133sollte auch hier wieder deshalb lokal begrenzt durchgeführt werden, damit die das Sicherheitselement 133 kennzeichnende Flexibilität im Bereich um die Verbindungsstelle 115 aufrechterhalten werden kann bzw. gewährleistet bleibt. Eine lokal applizierte Verstreckung im proximalen Sicherheitselementabschnitts 137 in der vorausgehend beschriebenen Form kann entweder allein oder aber auch ergänzend zu einem Stoffschluss, der beispielsweise als Verklebung ausgeführt ist, durchgeführt werden.

In Fig. 36a ist der distale Sicherheitselementabschnitt 135 als Formschluss ausgeführt und an seinem distalen Ende zusätzlich als Stoffschluss radial innen mit der Nadelspitze 71 verklebt. Der proximale Sicherheitselementabschnitt 137 ist ebenfalls als Stoffschluss ausgeführt und radial innen mit dem Leiter 61 verklebt. In Fig. 36b erstreckt sich der proximale Sicherheitselementabschnitt 137 relativ lang proximalwärts, vorzugsweise über die gesamte axiale Länge des Leiters 61, sodass ein ausreichender Reib-/Kraftschluss mit dem Leiter 61 gegeben ist und es keiner Verklebung des proximalen Sicherheitselementabschnitts 137 mit dem Leiter 61 bedarf. Die Länge des axialen Abstands 134 kann dadurch nicht eindeutig festgelegt werden, sondern ist stattdessen fließend.

Insbesondere bei den schlauchförmigen Ausführungsbeispielen gemäß Fig. 36a-c kann es vorteilhaft sein, wenn das Stabilisierungselement 123 (in Fig. 36a-c nicht gezeigt) zumindest teilweise das Sicherheitselement 133 oder umgekehrt das Sicherheitselement 133 zumindest teilweise das Stabilisierungselement 123 (in Fig. 36a-c nicht gezeigt) umschließen. Vorzugsweise umschließt das Sicherheitselement 133 das Stabilisierungselement 123, da das innenseitige Stabilisierungselement 123 dann möglichst großflächig mit seiner radialen Innenseite mit der Nadelspitze 71 und dem distalen Ende des Einführabschnitts 11 verklebt sein kann. Das außenseitige Sicherheitselement 133 kann dabei an seinen axialen Enden verklebt sein und einen axial zwischen den verklebten Enden angeordneten mittleren Abschnitt aufweisen, der nicht verklebt ist.

In Fig. 36c ist das Sicherheitselement 133 in Form eines distal geschlossenen Schlauchs als auswechselbarer steriler Einwegartikel von distal über die gesamte Nadelspitze 71 gestülpt. Der Schlauch kann dabei beispielsweise als lichttransparenter Schrumpfschlauch thermisch aufgeschrumpft sein und/oder elastisch dehnbar über die gesamte Nadelspitze 71 gezogen. Es bedarf dann ggf. gar keiner Verklebung des Sicherheitselements 133, wenn ein innenseitiger Reib-/Kraftschluss ausreicht, um die Nadelspitze 71 vor einem vollständigen Abriss bzw. Abbruch zu sichern.

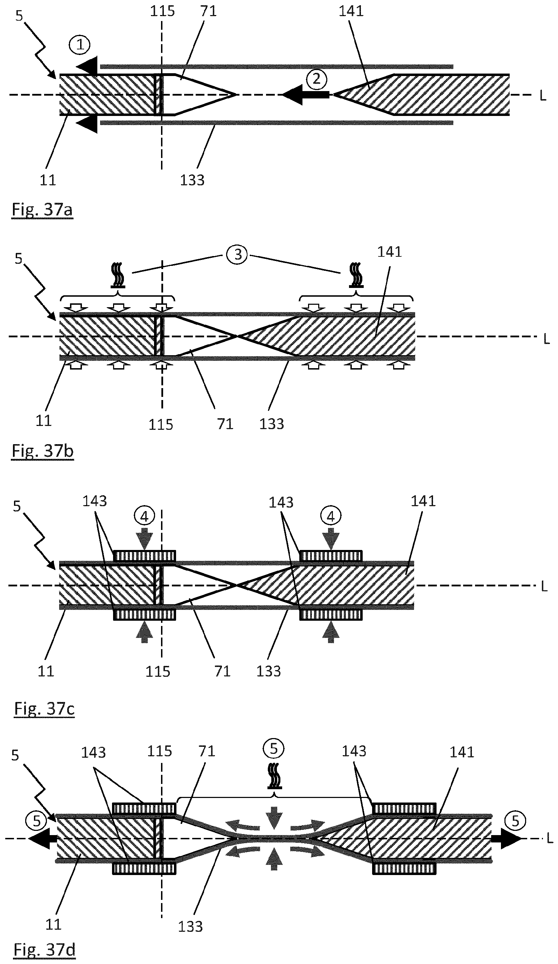

In Fig. 37a-d ist beispielhaft gezeigt, wie eine auf einen definierten axialen Abschnitt begrenzte axiale Verstreckung oder Nachverstreckung des Sicherheitselements 133 direkt am Lichtapplikator 5 erfolgen kann. Der anfangs über seine gesamte Länge zylinderförmige Schrumpfschlauch 133 wird in einem ersten Schritt (Nummern der Schritte sind eingekreist) über das distale Ende des Lichtapplikators 5 geschoben, und zwar derart, dass er distal übersteht. In einem zweiten Schritt wird eine Hilfsform 141, deren Außenkontur beispielsweise derjenigen des Lichtapplikators 5 entspricht, entlang der Längsachse L nah an die Spitze des Lichtapplikators 5 proximalwärts herangeführt, siehe Fig. 37a.

In einem dritten Schritt (s. Fig. 37b) wird unter Zufuhr von Wärme, die lediglich in den zylindrischen Abschnitten des Lichtapplikators 5 und der Hilfsform 141 appliziert wird, der Schrumpfschlauch 133 in diesen Abschnitten auf die dortigen äußeren Abmessungen von Lichtapplikator 5 und Hilfsform 141 zusammengeschrumpft. In einem vierten Schritt (s. Fig. 37c) wird der Schrumpfschlauch 133 mit Hilfe von spannzangenartigen Hilfsvorrichtungen 143 großflächig und radial umlaufend an die zylindrischen Endbereiche von Lichtapplikator 5 und Hilfsform 141 derart angedrückt, dass der Schrumpfschlauch 133 bei Zugbelastung in axiale Richtung auf dem Lichtapplikator 5 und auf der Hilfsform 141 nicht verrutschen kann. Hierbei umfasst eine Hilfsvorrichtung 143 die Verbindungsstelle 115 am Lichtapplikator 5. In einem fünften Schritt (s. Fig. 37d) werden der Lichtapplikator 5 und die Hilfsform 141 entlang der Längsachse L voneinander wegbewegt. Dies geschieht wieder unter Zufuhr von Wärme, deren Applikation nun aber auf den Bereich zwischen den beiden Hilfsvorrichtungen 143 begrenzt wird. Die resultierende Temperatur am Schrumpfschlauch 133 sollte dabei über der Glastemperatur des eingesetzten Schlauchmaterials liegen.

Das Erhitzen und gleichzeitige axiale Strecken der Molekülketten des Schrumpfschlauches 133 im Bereich zwischen den Hilfsvorrichtungen 143 bewirkt erstens eine Verjüngung des bis zum Ende des vierten Schritts (Fig. 37c) noch immer zylinderförmigen Schrumpfschlauches 133 und damit eine Adaption des Schrumpfschlauches 133 an die Nadelspitze 71, wie in Fig. 36d gezeigt, und zweitens eine Zunahme der Steifigkeit und daraus resultierend eine Zunahme der Formstabilität des Schrumpfschlauches 133 im axial verstreckten Bereich zwischen den beiden Hilfsvorrichtungen 143. Die Fixierung des Schrumpfschlauches 133 am zylindrischen Teil des Lichtapplikators 5 mittels der Hilfsvorrichtung 143 um die Verbindungsstelle 115, die weitgehende Begrenzung der Wärmeapplikation auf den Bereich zwischen den beiden Hilfsvorrichtungen 143 während des Ziehvorganges und die Form von Lichtapplikator 5 und Hilfsform 141 bewirken, dass die Verstreckung des Schrumpfschlauchs 133 (mit Verjüngung einerseits und Zunahme der Steifigkeit und Formstabilität andererseits) in der gewünschten Weise auf den Bereich zwischen den beiden Hilfsvorrichtungen 143, also die konischen Bereiche, begrenzt bleibt. In den von den Hilfsvorrichtungen 143 umfassten Bereichen findet dabei keine Verstreckung bzw. Nachverstreckung statt und der Schrumpfschlauch 133 behält dort in vorteilhafter Weise seine das Sicherheitselement 133 kennzeichnende Flexibilität. Es wird lokal, also im Bereich zwischen den beiden Hilfsvorrichtungen 143, so viel Wärme appliziert und dadurch eine so hohe Temperatur am Schrumpfschlauch 133 erzeugt, dass die Veränderungen thermisch fixiert werden. Nach dem Verstrecken wird in einem letzten Schritt (nicht gezeigt) der Schrumpfschlauch 133 an der gewünschten Stelle im konischen Bereich der Nadelspitze 71 bzw. der formgleichen Aufbauhilfe durchtrennt, vorzugsweise an derjenigen Stelle, die am stärksten verjüngt ist.

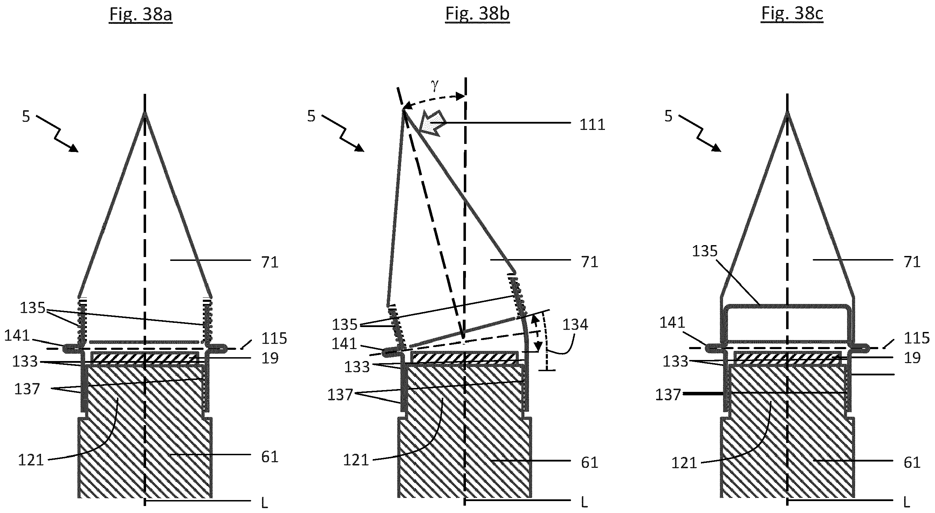

In Fig. 38a-c ist eine Ausführungsform gezeigt, bei der das Sicherheitselement 133 in Form von mindestens einer schnur- oder fadenförmigen Komponente gebildet ist, die eine schlaufenartig geführte Überschusslänge 145 aufweist. Vorzugsweise befindet sich die schlaufenartig geführte Überschusslänge 145 in einem axial mittleren Abschnitt zwischen zwei verklebten axialen Endbereichen des Sicherheitselements 133 und umgibt so die Verbindungsstelle 115. Die schlaufenartig geführte Überschusslänge 145 ist vorzugsweise selbst nicht verklebt, sondern kann im Fall eines Bruchs oder Abrisses der Nadelspitze 71 bei weiteren Krafteinwirkungen 111 auf die Nadelspitze 71 resilient nachgeben, um so die erforderliche Flexibilität des Sicherheitselementes 133 im Bedarfsfall verfügbar bzw. nutzbar machen zu können. Die Überschusslänge 145 ist insbesondere dann sinnvoll, wenn für das Sicherheitselement 133 ein Material mit einer relativ kurzen maximalen Dehnlänge gewählt werden soll. Bei der vorliegenden Ausführungsform bestimmt also diese schlaufenartig geführte Überschusslänge 145 maßgeblich den maximal möglichen axialen Abstand 134 zwischen dem distalen Sicherheitselementabschnitt 135 und dem proximalen Sicherheitselementabschnitt 137, also denjenigen axialen Abstand 134, der die kennzeichnende Flexibilität des Sicherheitselements 133 bestimmt. Außerdem kann durch die Überschusslänge eine größere radiale Innenfläche des Sicherheitselement 133 mit der Nadelspitze 71 und dem Leiter 61 verklebt bzw. verlötet werden. In Fig. 38a ist das Sicherheitselement 133 mehrteilig, hier zweiteilig, als sich axial erstreckende Fäden oder Schnüre ausgebildet. Die Überschusslänge 145 muss dabei nicht wie gezeigt radial abstehen, sondern ist vorzugsweise radial anliegend schlaufenartig gefaltet. Im Falle eines wie in Fig. 37b gezeigten Abrisses der Nadelspitze 71, kann sich die Überschusslänge 145 ausfalten, sodass die Nadelspitze 71 um einen Verbiegungswinkel $\gamma$ der Kraft 111 resilient nachgeben kann, d.h. $\gamma \geq 20°$, ohne vollständig abzureißen. Optional können weitere Sicherheitselemente 133 am Lichtapplikator 5, beispielsweise in einer Ebene senkrecht zur Bildebene, angebracht sein.

In Fig. 38c ist das Sicherheitselement 133 einteilig als ein Faden oder eine Schnur ausgestaltet, der oder die sich U-förmig durch die Nadelspitze 71 erstreckt. Das Sicherheitselement 133 ist also zum Teil in die Nadelspitze 71 eingebettet bzw. von dieser umgossen. Dadurch wird ein zusätzlicher Formschluss mit der Nadelspitze 71 erreicht. Optional kann der Lichtapplikator 5 mit mehreren einteiligen Sicherheitselementen 133 aus der Fig. 38c bestückt sein. Beispielsweise kann ein zweites Sicherheitselement 133 in einer Ebene senkrecht zur Bildebene der Fig. 38c angeordnet sein (nicht dargestellt).

Die nummerierten Bezeichnungen der Bauteile oder Bewegungsrichtungen als "erste", "zweite", "dritte" usw. sind hierin rein willkürlich zur Unterscheidung der Bauteile oder Bewegungsrichtungen untereinander gewählt und können beliebig anders gewählt werden. Es ist damit kein Bedeutungsrang verbunden. Eine Bezeichnung eines Bauteils oder technischen Merkmals als "erstes" soll nicht dahingehend missverstanden werden, dass es ein zweites Bauteil oder technisches Merkmal dieser Art geben muss. Außerdem können etwaige Verfahrensschritte, soweit nicht explizit anders erläutert oder zwingend erforderlich, in beliebiger Reihenfolge und/oder zeitlich teilweise oder ganz überlappend durchgeführt werden.

Bezugszeichenliste:

| | |
|---|---|
| 1 | Lichtapplikatorsystem |
| 3 | Lichtapplikatorbetriebseinheit |
| 5 | Lichtapplikator |

| | |
|---|---|
| 7 | Kabelabschnitt des Lichtapplikators |
| 9 | Stecker des Lichtapplikators |
| 11 | Einführabschnitt des Lichtapplikators |
| 13 | Haut |
| 15 | gesundes Gewebe |
| 17 | pathologisches Gewebe |
| 19 | LED |
| 21 | Anschluss der Lichtapplikatorbetriebseinheit |
| 23 | Versorgungsmodul |
| 25 | Steuerungsmodul |
| 27 | Organ |
| 29 | Flachbandkabel |
| 31 | Arbeitskanal |
| 33 | Schaft |
| 35 | Schaftinstrument |
| 37 | Einführabschnitt des Endoskops |
| 39 | Bildsensor |
| 41 | erster Schaftabschnitt |
| 43 | zweiter Schaftabschnitt |
| 45 | dritter Schaftabschnitt |
| 49, 49a, 49b | Ummantelung des Lichtapplikators |
| 50 | erster elektrischer Kontakt der LED |
| 51 | zweiter elektrischer Kontakt der LED |
| 53 | Adapterstück |
| 55 | Metallhälfte |
| 57 | Metallhälfte |
| 59 | Isolator-Schicht |
| 61 | elektrischer Leiter |
| 61a | erster elektrischer Leiter |
| 61b | zweiter elektrischer Leiter |
| 63 | Kontaktaufnahme |
| 65 | Kontaktaufnahme |
| 67 | Hülse |
| 69 | Kontaktbügel |
| 71 | Nadelspitze / Streukörper |
| 73 | Verbindungsanschluss |
| 74 | Kern des ersten elektrischen Leiters |
| 75 | proximaler Nadelspitzenabschnitt bzw. erste Wärmedämmschicht |
| 76 | Mantel des ersten elektrischen Leiters |
| 77 | zweite Wärmedämmschicht |
| 79 | dritte Wärmedämmschicht |
| 80 | Vertiefung |
| 81 | Stirnfläche des Streukörpers |
| 82 | Aufweitung |
| 83 | Kante des Streukörpers |
| 84 | Hinterschnitt |
| 85 | Spitze des Streukörpers |
| 87 | lichtstreuendes Material |
| 89 | von der LED abgestrahltes Licht |
| 91 | vom Streukörper abgestrahltes Licht |
| 93 | Nadelspitzenendbereich des Streukörpers |
| 95 | Verstärkungselement |
| 97 | Kanten des Streukörpers |
| 99 | Schlitze im Verstärkungselement |
| 101 | Schneidkanten des Verstärkungselements |
| 103 | Spitze des Verstärkungselements |
| 105 | erster Streukörperabschnitt |
| 107 | zweiter Streukörperabschnitt |
| 109 | dritter Streukörperabschnitt |
| 111 | seitliche Krafteinwirkung |
| 113 | externer Körper |
| 115 | Verbindungsstelle |
| 117 | axiale Zugkraft |
| 119 | proximalseitiger Hülsenfortsatz |
| 121 | distaler Endabschnitt des Leiters |
| 123 | Stabilisierungselement |
| 125 | proximaler Nadelspitzenabschnitt |
| 127 | distaler Stabilisierungselementabschnitt |
| 129 | Durchbrechungen |
| 131 | distaler Nadelspitzenabschnitt |
| 133 | Sicherheitselement |
| 134 | axialer Abstand |
| 135 | distaler Sicherheitselementabschnitt |
| 137 | proximaler Sicherheitselementabschnitt |
| 139 | Dehnlänge |
| 141 | Hilfsform |
| 143 | Hilfsvorrichtungen |
| 145 | Überschusslänge |
| A | erster Lichtapplikatorabschnitt |
| B | zweiter Lichtapplikatorabschnitt |
| C | dritter Lichtapplikatorabschnitt |
| D | vierter Lichtapplikatorabschnitt |
| L | Längsachse des Lichtapplikators |
| S1 | erste Längsschnittebene |
| S1 | zweite Längsschnittebene |
| $\alpha$ | erster Winkel |
| $\beta$ | zweiter Winkel |
| $\gamma$ | Verbiegungswinkel |

**Patentansprüche**

1. Lichtapplikator (5) zur Untersuchung und/oder Behandlung von einem organischen Körper, wobei der Lichtapplikator (5) einen minimalinvasiven Einführabschnitt (11) aufweist, der sich entlang einer axialen Längsrichtung (L) erstreckt und an seinem distalen Ende eine LED (19) aufweist, wobei am distalen Ende des Einführabschnitts (11) eine sich zumindest teilweise distalwärts von der LED (19) erstreckende und sich distalwärts verjüngende Nadelspitze (71) mit einem lichttransparenten Streukörper zur Streuung des Lichts der LED (19) angeordnet ist, wobei ein zumindest teilweise umfangseitig das distale Ende des Einführabschnitts (11) und zumindest teilweise umfangseitig die Nadelspitze (71) umgebendes Sicherheitselement (133) mit dem Einführabschnitt (11) und der Nadelspitze (71) verbunden ist, wobei das Sicherheitselement (133) zumindest abschnittsweise flexibel ist und im Falle eines zumindest teilweisen Abrisses oder Bruchs der Nadelspitze (71) diese am distalen Ende des Einführabschnitts (11) vor einem vollständigen Abreißen vom Lichtapplikator (5) sichert.

2. Lichtapplikator (5) nach Anspruch 1, wobei das Sicherheitselement (133) mittels einer ersten Verbindung mit dem distalen Ende des Einführabschnitts (11) und mittels einer zweiten Verbindung mit der Nadelspitze (71) jeweils stoff-, kraft-/reib- und/oder formschlüssig verbunden ist, wobei die zweite Verbindung einen axialen Abstand (134) in Längsrichtung (L) von der ersten Verbindung hat.

3. Lichtapplikator (5) nach Anspruch 2, wobei der Abstand (134) mehr als 50% der axialen Länge des Sicherheitselements (133) beträgt.

4. Lichtapplikator (5) nach einem der vorhergehenden Ansprüche, wobei das Sicherheitselement (133) als geformter Metalldraht ausgeführt ist, der zumindest abschnittsweise formschlüssig mit der Nadelspitze (71) verbunden ist.

5. Lichtapplikator (5) nach einem der vorhergehenden Ansprüche, wobei das Sicherheitselement (133) zumindest abschnittsweise axial verstreckt ist.

6. Lichtapplikator (5) nach einem der vorhergehenden Ansprüche, wobei das Sicherheitselement (133) einen radial mittig angeordneten distalen Sicherheitselementabschnitt (135) und einen radial außenseitig angeordneten proximalen Sicherheitselementabschnitt (137) aufweist, wobei der proximale Sicherheitselementabschnitt (137) mit dem distalen Ende des Einführabschnitts (11) verbunden ist und der distale Sicherheitselementabschnitt (135) ein radial mittiges Verstärkungselement (95) in Form einer Metallspitze der Nadelspitze (71) bildet.

7. Lichtapplikator (5) nach einem der vorhergehenden Ansprüche, wobei das Sicherheitselement (133) in Form eines distal geschlossenen Schlauchs als auswechselbarer steriler Einwegartikel von distal über die gesamte Nadelspitze (71) stülpbar ist.

8. Lichtapplikator (5) nach einem der vorhergehenden Ansprüche, wobei das Sicherheitselement (133) in Form von mindestens einer schnur- oder fadenförmigen Komponente gebildet ist, die eine schlaufenartig geführte Überschusslänge (141) aufweist.

9. Lichtapplikator (5) nach einem der vorhergehenden Ansprüche, wobei ein umfangseitig das distale Ende des Einführabschnitts (11) und die Nadelspitze (71) zumindest teilweise umgebendes Stabilisierungselement (123) mit dem Einführabschnitt (11) und der Nadelspitze (71) verbunden ist, wobei das Stabilisierungselement (123) ein Material aufweist, das zumindest in Längsrichtung (L) axial verstreckt ist.

10. Lichtapplikator (5) nach Anspruch 8, wobei das Stabilisierungselement (123) zumindest teilweise das Sicherheitselement (133) oder das Sicherheitselement (133) zumindest teilweise das Stabilisierungselement (123) umschließt.

11. Lichtapplikator (5) nach Anspruch 8 oder 9, wobei das Stabilisierungselement (123) und das Sicherheitselement (133) abwechselnd in einer zur Längsrichtung (L) orthogonalen Umfangsrichtung (U) das distale Ende des Einführabschnitts (11) und die Nadelspitze (71) zumindest teilweise umgreifen.

12. Lichtapplikator (5) nach einem der vorhergehenden Ansprüche, wobei das Stabilisierungselement (123) und/oder das Sicherheitselement (133) das distale Ende des Einführabschnitts (11) und die Nadelspitze (71) zumindest teilweise schlauchförmig umgreift.

13. Lichtapplikator (5) nach einem der vorhergehenden Ansprüche, wobei das Stabilisierungselement (123) und/oder das Sicherheitselement (133) zumindest teilweise aus einer lichttransparenten Kunststofffolie besteht.

14. Lichtapplikator (5) nach einem der vorhergehenden Ansprüche, wobei das Stabilisierungselement (123) und/oder das Sicherheitselement (133) eine axiale Verbindungsstelle (115) zwischen dem distalen Ende des Einführabschnitts (11) und der Nadelspitze (71) vollständig umlaufend umschließt.

15. Lichtapplikator (5) nach einem der vorhergehenden Ansprüche, wobei das Stabilisierungselement (123) und/oder das Sicherheitselement (133) eine radiale Dicke aufweist, die weniger als 20% des Durchmessers des Einführabschnitts (11) beträgt.

16. Lichtapplikator (5) nach einem der vorhergehenden Ansprüche, wobei das Stabilisierungselement (123) einen distalen Stabilisierungselementabschnitt (127) und/oder das Sicherheitselement (133) einen distalen Sicherheitselementabschnitt (135) aufweist, wobei der distale Stabilisierungselementabschnitt (127) und/oder der distale Sicherheitselementabschnitt (135) an der Nadelspitze (71) zumindest teilweise radial zuläuft.

17. Lichtapplikator (5) nach Anspruch 15, wobei der distale Stabilisierungselementabschnitt (127) und/oder der distale Sicherheitselementabschnitt (135) zumindest teilweise in der Nadelspitze (71) eingegossen und/oder umspritzt ist.

18. Lichtapplikator (5) nach einem der vorhergehenden Ansprüche, wobei das Stabilisierungselement (123) und/oder das Sicherheitselement (133) zumindest teilweise das distale Ende des Einführabschnitts (11) und zumindest einen proximalen Nadelspitzenabschnitt (125) strumpfförmig umgreift, wobei das Stabilisierungselement (123) und/oder das Sicherheitselement (133) axiale Durchbrechungen (129) aufweist, durch welche ein distaler Nadelspitzenabschnitt (131) mit dem proximalen Nadelspitzenabschnitt (125) stoffschlüssig verbunden ist.

19. Lichtapplikator (5) nach einem der Ansprüche 8 bis 17, wobei das Stabilisierungselement (123) axial in Längsrichtung (L) eine höhere Zugfestigkeit aufweist als in eine zur Längsrichtung (L) orthogonale Umfangsrichtung (U).

20. Lichtapplikator (5) nach einem der Ansprüche 8 bis 18, wobei das Stabilisierungselement (123) zumindest teilweise aus einem kristallisierten oder teilkristallisierten Polymer besteht, dessen Molekülketten eine Vorzugsausrichtung in Längsrichtung (L) aufweisen.

21. Lichtapplikator (5) nach einem der Ansprüche 8 bis 19, wobei die Zugfestigkeit des Stabilisierungselements (123) in Längsrichtung (L) mindestens doppelt so hoch ist wie die Zugfestigkeit des Stabilisierungselements (123) in einer zur Längsrichtung (L) orthogonalen Umfangsrichtung (U).

22. Lichtapplikator (5) nach einem der Ansprüche 8 bis 20, wobei das Stabilisierungselement (123) als ein proximalseitiger Nadelspitzenhülsenfortsatz (119) ausgebildet ist, wobei der Nadelspitzenhülsenfortsatz (119) einen Faser-Kunststoff-Verbund mit axial verstreckten Fasern aufweist, wobei die Fasern eine Vorzugsausrichtung in Längsrichtung (L) aufweisen.

23. Lichtapplikator (5) nach einem der Ansprüche 8 bis 21, wobei das Stabilisierungselement (123) über mehr als 50% seiner axialen Länge radial innenseitig jeweils stoff-, kraft-/reib- oder formschlüssig verbunden ist.

24. Verfahren zum Herstellen eines Lichtapplikators (5) mit den Schritten:

- Bereitstellen eines Lichtapplikators (5) mit einem minimalinvasiven Einführabschnitt (11), der sich entlang einer axialen Längsrichtung (L) erstreckt und an seinem distalen Ende eine LED (19) aufweist, wobei am distalen Ende des Einführabschnitts (11) eine sich zumindest teilweise distalwärts von der LED (19) erstreckende und sich distalwärts verjüngende Nadelspitze (71) mit einem lichttransparenten Streukörper zur Streuung des Lichts der LED (19) angeordnet ist,
- Aufziehen eines flexiblen Sicherheitselements (133) in Form eines Schrumpfschlauchs über die Nadelspitze (71) und zumindest teilweise über das distale Ende des Einführabschnitts (11),
- Aufschrumpfen des Sicherheitselements (133) auf das distale Ende des Einführabschnitts (11), und
- Axiales Verstrecken eines die sich verjüngende Nadelspitze (71) umgebenden axialen Abschnitts des Sicherheitselements (133).

25. Verfahren nach Anspruch 24, wobei während des axialen Verstreckens das Sicherheitselement (133) zumindest abschnittsweise am distalen Ende des Einführabschnitts (11) festgehalten wird und somit dort unverstreckt und flexibel bleibt.

26. Verfahren nach Anspruch 24 oder 25, wobei während des axialen Verstreckens oder in einem separaten Schritt das Sicherheitselement zumindest abschnittsweise erwärmt wird, wodurch der die verjüngende Nadelspitze umgebende axiale Abschnitt des Sicherheitselements an die sich verjüngende Nadelspitze thermisch fixiert wird.

**Claims**

1. Light applicator (5) for the examination and/or treatment of an organic body, wherein the light applicator (5) has a minimally invasive insertion section (11), which extends along an axial longitudinal direction (L) and at its distal end has an LED (19), wherein at the distal end of the insertion section (11) is arranged a needle tip (71) extending at least partially distalwards from the LED (19), and tapering distalwards with a light-transparent scattering body for purposes of scattering the light of the LED (19), wherein a security element (133) at least partially peripherally surrounding the distal end of the insertion section (11), and at least partially peripherally surrounding the needle tip (71), is connected to the insertion section (11) and the needle tip (71), wherein the security element (133) is flexible, in at least some sections, and, in the event of an at least partial break-off or fracture of the needle tip (71), secures the latter at the distal end of the insertion section (11) against a complete break-off from the light applicator (5).

2. Light applicator (5) according to Claim 1, wherein the security element (133) is connected by means of a first connection to the distal end of the insertion section (11), and by means of a second connection to the needle tip (71), in each case in a materially-bonded, a force/frictional-locking, and/or a form-locking manner, wherein the second connection has an axial distance (134) in the longitudinal direction (L) from the first connection.

3. Light applicator (5) according to Claim 2, wherein the distance (134) is more than 50% of the axial length of the security element (133).

4. Light applicator (5) according to one of the preceding claims, wherein the security element (133) is designed as a shaped metal wire, which is connected to the needle tip (71) in a form-locking manner, in at least some sections.

5. Light applicator (5) according to one of the preceding claims, wherein the security element (133) is axially stretched, in at least some sections.

6. Light applicator (5) according to one of the preceding claims, wherein the security element (133) has a radially, centrally arranged, distal security element section (135), and a radially, outwardly arranged, proximal security element section (137), wherein the proximal security element section (137) is connected to the distal end of the insertion section (11), and the distal security element section (135) forms a radially central reinforcing element (95) in the form of a metal tip of the needle point (71).

7. Light applicator (5) according to one of the preceding claims, wherein the security element (133) in the form of a distally closed tube, as a replaceable sterile disposable article, can be pulled over the entire needle tip (71) from the distal end.

8. Light applicator (5) according to one of the preceding claims, wherein the security element (133) is designed in the form of at least one cord-like or thread-like component, which has a loopform routed surplus length (141).

9. Light applicator (5) according to one of the preceding claims, wherein a stabilisation element (123), at least partially surrounding the distal end of the insertion section (11) and the needle tip (71), is connected to the insertion section (11) and the needle tip (71), wherein the stabilisation element (123) features a material, which is axially stretched, in at least the longitudinal direction (L).

10. Light applicator (5) according to Claim 8, wherein the stabilisation element (123) at least partially encloses the security element (133), or the security element (133) at least partially encloses the stabilisation element (123).

11. Light applicator (5) according to Claim 8 or 9, wherein the stabilisation element (123) and the security element (133) alternately at least partially surround, in a peripheral direction (U) orthogonal to the longitudinal direction (L), the distal end of the insertion section (11) and the needle tip (71).

12. Light applicator (5) according to one of the preceding claims, wherein the stabilisation element (123), and/or the security element (133), at least partially surround/surrounds the distal end of the insertion section (11) and the needle tip (71) in a tubular manner.

13. Light applicator (5) according to one of the preceding claims, wherein the stabilisation element (123), and/or the security element (133), at least partially consist/s of a light-transparent plastic film.

14. Light applicator (5) according to one of the preceding claims, wherein the stabilisation element (123), and/or the security element (133), completely surround/s an axial connection point (115) between the distal end of the insertion section (11) and the needle tip (71).

15. Light applicator (5) according to one of the preceding claims, wherein the stabilisation element (123), and/or the security element (133), have/has a radial thickness, which is less than 20% of the diameter of the insertion section (11).

16. Light applicator (5) according to one of the preceding claims, wherein the stabilisation element (123) features a distal stabilisation element section (127), and/or the security element (133) features a distal security element section (135), wherein the distal stabilisation element section (127), and/or the distal security element section (135), run/s at least partially radially to the needle tip (71).

17. Light applicator (5) according to Claim 15, wherein the distal stabilisation element section (127), and/or the distal security element section (135) are/is at least partially moulded, and/or overmoulded, in the needle tip (71).

18. Light applicator (5) according to one of the preceding claims, wherein the stabilisation element (123), and/or the security element (133), at least partially surround/s the distal end of the insertion section (11) and at least a proximal needle tip section (125) in a stocking-shaped manner, wherein the stabilisation element (123), and/or the security element (133), have/has axial openings (129), through which a distal needle tip section (131) is connected to the proximal needle tip section (125) in a materiallybonded manner.

19. Light applicator (5) according to one of the Claims 8 to 17, wherein the stabilisation element (123) has a higher tensile strength axially in the longitudinal direction (L) than in a peripheral direction (U) orthogonal to the longitudinal direction (L).

20. Light applicator (5) according to one of the Claims 8 to 18, wherein the stabilisation element (123) consists, at least partially, of a crystallised, or partially crystallised, polymer, whose molecular chains have a preferred orientation in the longitudinal direction (L).

21. Light applicator (5) according to one of the Claims 8 to 19, wherein the tensile strength of the stabilisation element (123) in the longitudinal direction (L) is at least twice as high as the tensile strength of the stabilisation element (123) in a peripheral direction (U) orthogonal to the longitudinal direction (L).

22. Light applicator (5) according to one of the Claims 8 to 20, wherein the stabilisation element (123) is designed as a proximal needle tip sleeve extension (119), wherein the needle tip sleeve extension (119) features a fibre-plastic composite with axially stretched fibres, wherein the fibres have a preferred orientation in the longitudinal direction (L).

23. Light applicator (5) according to one of the Claims 8 to 21, wherein the stabilisation element (123) is connected over more than 50% of its axial length radially on the inside in a materiallybonded, a force/frictional-locking, and/or a form-locking, manner.

24. Method of production of a light applicator (5), with the steps:

    - Provision of a light applicator (5) with a minimally invasive insertion section (11), which extends along an axial longitudinal direction (L) and has an LED (19) at its distal end, wherein at the distal end of the insertion section (11), a needle tip (71) extending at least partially distalwards from the LED (19), and tapering distalwards, is arranged with a light-transparent scattering body for purposes of scattering the light of the LED (19),
    - Pulling of a flexible security element (133) in the form of shrink tubing over the needle tip (71) and at least partially over the distal end of the insertion section (11),
    - Shrinkage of the security element (133) onto the distal end of the insertion section (11), and
    - Axial stretching of an axial section of the security element (133) surrounding the tapering needle tip (71).

25. Method according to Claim 24, wherein during the axial stretching, the security element (133) is retained, in at least some sections, at the distal end of the insertion section (11), and thus there remains unstretched and flexible.

26. Method according to Claim 24 or 25, wherein during the axial stretching, or in a separate step, the security element is heated, in at least some sections, whereby the axial section of the security element surrounding the tapering needle tip is thermally fixed to the tapering needle tip.

**Revendications**

1. Applicateur de lumière (5) destiné à l'examen et/ou au traitement d'un corps organique, l'applicateur de lumière (5) comprenant une section d'introduction (11) minimalement-invasive qui s'étend le long d'une direction longitudinale axiale (L) et qui comprend une LED (19) à son extrémité distale, une pointe d'aiguille (71) s'étendant à partir de la LED (19) au moins partiellement en direction distale et se rétrécissant en direction distale et munie d'un corps de diffusion transparent à la lumière permettant de diffuser la lumière de la LED (19) étant agencée à l'extrémité distale de la section d'introduction (11), un élément de sécurité (133) entourant l'extrémité distale de la section d'introduction (11) au moins partiellement du côté de sa périphérie et entourant la pointe d'aiguille (71) au moins partiellement du côté de sa périphérie étant relié à la section d'introduction (11) et à la pointe d'aiguille (71), l'élément de sécurité (133) étant flexible au moins par sections et protégeant ledit élément de sécurité contre une séparation complète de l'applicateur de lumière (5) en cas de séparation ou de rupture au moins partielle de la pointe d'aiguille (71) à l'extrémité distale de la section d'introduction (11).

2. Applicateur de lumière (5) selon la revendication 1, l'élément de sécurité étant relié, respectivement par complémentarité de matière, de force/de friction et/ou de forme, à l'extrémité distale de la section d'introduction (11) à l'aide d'une première liaison et à la pointe d'aiguille (71) à l'aide d'une seconde liaison, la seconde liaison ayant un espacement axial par rapport à la première liaison dans la direction longitudinale (L).

3. Applicateur de lumière (5) selon la revendication 2, l'espacement (134) étant supérieur à 50 % de la longueur axiale de l'élément de sécurité (133).

4. Applicateur de lumière (5) selon l'une quelconque des revendications précédentes, l'élément de sécurité (133) étant réalisé sous la forme d'un fil métallique mis en forme qui est relié à la pointe d'aiguille (71) par complémentarité de forme au moins par sections.

**5.** Applicateur de lumière (5) selon l'une quelconque des revendications précédentes, l'élément de sécurité (133) étant étiré de manière axiale au moins par sections.

**6.** Applicateur de lumière (5) selon l'une quelconque des revendications précédentes, l'élément de sécurité (133) comprenant une section d'élément de sécurité distale (135) agencée radialement au centre et une section d'élément de sécurité proximale (137) agencée radialement à l'extérieur, la section d'élément de sécurité proximale (137) étant reliée à l'extrémité distale de la section d'introduction (11) et la section d'élément de sécurité distale (135) formant un élément de renforcement (95) radialement central sous la forme d'une pointe métallique de la pointe d'aiguille (71).

**7.** Applicateur de lumière (5) selon l'une quelconque des revendications précédentes, l'élément de sécurité (133) pouvant être enfilé depuis une position distale sur toute la pointe d'aiguille (71) sous la forme d'un tuyau fermé de manière distale faisant office d'article jetable stérile remplaçable.

**8.** Applicateur de lumière (5) selon l'une quelconque des revendications précédentes, l'élément de sécurité (133) étant réalisé sous la forme d'au moins un composant en forme de cordon ou de fil qui comprend une longueur excédentaire (141) guidée pour adopter la forme d'une boucle.

**9.** Applicateur de lumière (5) selon l'une quelconque des revendications précédentes, un élément de stabilisation (123) entourant au moins partiellement l'extrémité distale de la section d'introduction (11) et la pointe d'aiguille (71) sur le côté périphérique étant relié à la section d'introduction (11) et à la pointe d'aiguille (71), l'élément de stabilisation (123) comprenant un matériau qui est axialement étiré au moins dans la direction longitudinale (L).

**10.** Applicateur de lumière (5) selon la revendication 8, l'élément de stabilisation (123) entourant au moins partiellement l'élément de sécurité (133) ou l'élément de sécurité (133) entourant au moins partiellement l'élément de stabilisation (123).

**11.** Applicateur de lumière (5) selon la revendication 8 ou 9, l'élément de stabilisation (123) et l'élément de sécurité (133) entourant au moins partiellement l'extrémité distale de la section d'introduction (11) et la pointe d'aiguille (71) de manière alternée dans une direction périphérique (U) orthogonale par rapport à la direction longitudinale (L).

**12.** Applicateur de lumière (5) selon l'une quelconque des revendications précédentes, l'élément de stabilisation (123) et/ou l'élément de sécurité (133) entourant l'extrémité distale de la section d'introduction (11) et la pointe d'aiguille (71) au moins partiellement en adoptant la forme d'un tuyau.

**13.** Applicateur de lumière (5) selon l'une quelconque des revendications précédentes, l'élément de stabilisation (123) et/ou l'élément de sécurité (133) étant constitué(s) au moins partiellement d'un film plastique transparent à la lumière.

**14.** Applicateur de lumière (5) selon l'une quelconque des revendications précédentes, l'élément de stabilisation (123) et/ou l'élément de sécurité (133) enfermant complètement de manière périphérique une jonction axiale (115) entre l'extrémité distale de la section d'introduction (11) et la pointe d'aiguille (71).

**15.** Applicateur de lumière (5) selon l'une quelconque des revendications précédentes, l'élément de stabilisation (123) et/ou l'élément de sécurité (133) ayant une épaisseur radiale inférieure à 20 % du diamètre de la section d'introduction (11).

**16.** Applicateur de lumière (5) selon l'une quelconque des revendications précédentes, l'élément de stabilisation (123) comprenant une section d'élément de stabilisation distale (127) et/ou l'élément de sécurité (133) comprenant une section d'élément de sécurité distale (135), la section d'élément de stabilisation distale (127) et/ou la section d'élément de sécurité distale (135) se rétrécissant de manière au moins partiellement radiale à la pointe d'aiguille (71).

**17.** Applicateur de lumière (5) selon la revendication 15, la section d'élément de stabilisation distale (127) et/ou la section d'élément de sécurité distale (135) étant au moins partiellement moulée(s) et/ou surmoulée(s) dans la pointe d'aiguille (71).

**18.** Applicateur de lumière (5) selon l'une quelconque des revendications précédentes, l'élément de stabilisation (123) et/ou l'élément de sécurité (133) entourant au moins partiellement l'extrémité distale de la section d'introduction (11) et au moins une section de pointe d'aiguille proximale (125) en adoptant une forme de chaussette, l'élément de stabilisation (123) et/ou l'élément de sécurité (133) comprenant des percements axiaux (129) à travers lesquels une section de pointe d'aiguille distale (131) est reliée par complémentarité de matière à la section de pointe d'aiguille proximale (125).

**19.** Applicateur de lumière (5) selon l'une quelconque des revendications 8 à 17, l'élément de stabilisation (123) ayant, de manière axiale dans la direction longitudinale (L), une résistance à la traction plus élevée que dans une direction périphérique (U) orthogonale par rapport à la direction longitudinale (L).

20. Applicateur de lumière (5) selon l'une quelconque des revendications 8 à 18, l'élément de stabilisation (123) étant constitué au moins partiellement d'un polymère cristallisé ou partiellement cristallisé dont les chaînes moléculaires ont un alignement préférentiel dans la direction longitudinale (L).

21. Applicateur de lumière (5) selon l'une quelconque des revendications 8 à 19, la résistance à la traction de l'élément de stabilisation (123) dans la direction longitudinale (L) étant au moins égale à deux fois la résistance à la traction de l'élément de stabilisation (123) dans une direction périphérique (U) orthogonale par rapport à la direction longitudinale (L).

22. Applicateur de lumière (5) selon l'une quelconque des revendications 8 à 20, l'élément de stabilisation (123) étant réalisé sous la forme d'un prolongement de manchon de pointe d'aiguille (119) situé du côté proximal, le prolongement de manchon de pointe d'aiguille (119) comprenant un composite fibre-plastique avec des fibres étirées axialement, les fibres ayant une orientation préférentielle dans la direction longitudinale (L).

23. Applicateur de lumière (5) selon l'une quelconque des revendications 8 à 21, l'élément de stabilisation (123) étant relié radialement par complémentarité de matière, de force/de friction ou de forme à son côté intérieur sur plus de 50 % de sa longueur axiale.

24. Procédé de fabrication d'un applicateur de lumière (5), comprenant les étapes consistant à :

    - préparer un applicateur de lumière (5) avec une section d'introduction (11) mini-invasive qui s'étend le long d'une direction longitudinale axiale (L) et comprend une LED (19) à son extrémité distale, une pointe d'aiguille (71) s'étendant au moins partiellement en direction distale de la LED (19) et se rétrécissant en direction distale et munie d'un corps de diffusion transparent à la lumière permettant de diffuser la lumière de la LED (19) étant agencée à l'extrémité distale de la section d'introduction (11),
    - enfiler un élément de sécurité flexible (133) sous la forme d'un boyau rétractable sur la pointe de l'aiguille (71) et au moins en partie sur l'extrémité distale de la section d'introduction (11),
    - rétracter l'élément de sécurité (133) sur l'extrémité distale de la section d'introduction (11), et
    - étirer axialement une partie axiale de l'élément de sécurité (133) entourant la pointe d'aiguille (71) conique.

25. Procédé selon la revendication 24, pendant l'étirement axial, l'élément de sécurité (133) étant maintenu au moins par sections à l'extrémité distale de la section d'introduction (11) et restant ainsi non étiré et flexible.

26. Procédé selon la revendication 24 ou 25, pendant l'étirement axial ou dans une étape séparée, l'élément de sécurité étant chauffé au moins par sections, ce qui immobilise de manière thermique la partie axiale, entourant la pointe d'aiguille conique, de l'élément de sécurité au niveau de la pointe d'aiguille conique.

## Fig. 1

EP 3 906 835 B1

Fig. 2

EP 3 906 835 B1

EP 3 906 835 B1

## Fig. 3

Fig. 4

EP 3 906 835 B1

## Fig. 5

5

9

49    49    49    49

D    A    B    C

EP 3 906 835 B1

# Fig. 6

Fig. 7

Fig. 8

## Fig. 9a

## Fig. 9b

EP 3 906 835 B1

## Fig. 10a

61a

74
76
19
19

## Fig. 10b

61a

74
76
19
a

EP 3 906 835 B1

Fig. 11a

Fig. 11b

EP 3 906 835 B1

Fig. 11c

G          F          E

S 11

19

61          79    77          80    75    84          71

Fig. 11d

G          F          E

S 11

19

61          79    77          84    75    82          71

EP 3 906 835 B1

Fig. 12a    Fig. 12b    Fig. 12c

Fig. 13

EP 3 906 835 B1

Fig. 14

Fig. 15

Fig. 16

Fig. 17

Fig. 18

Fig. 19

EP 3 906 835 B1

Fig. 20

EP 3 906 835 B1

Fig. 21

Fig. 22

Fig. 23

EP 3 906 835 B1

Fig. 24

Fig. 25

Fig. 26

Fig. 27

EP 3 906 835 B1

Fig. 28a

Fig. 28b

Fig. 29

Fig. 30b

Fig. 30c

Fig. 30a

Fig. 31

71
123
2
121
119
61
L
5
11

Fig. 32a

Fig. 32b

Fig. 33b

Fig. 33a

Fig. 34a

Fig. 34b

Fig. 35a

Fig. 35b

EP 3 906 835 B1

Fig. 36a

Fig. 36b

Fig. 36c

Fig. 37a

Fig. 37b

Fig. 37c

Fig. 37d

EP 3 906 835 B1

Fig. 38a  Fig. 38b  Fig. 38c

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2449994 A1